# EUROPEAN PATENT APPLICATION

(11) **EP 4 628 497 A1**
(43) Date of publication of application: **08.10.2025**
(21) Application number: 24741299.2
(22) Date of filing: 10.01.2024
(51) Int. Cl.: C07J 43/00, A61K 48/00, A61K 31/56

(54) **STEROID-CATIONIC LIPID COMPOUND AND USE THEREOF**

(30) Priority: 10.01.2023 CN 202310034590
(71) Applicant: Jenkem Technology Co., Ltd. (Liaoning), Panjin, Liaoning 124000 (CN); Cansino (Shanghai) Biological Research Co., Ltd., Shanghai 201203 (CN)
(72) Inventor: YAN, Shengyong, Panjin, Liaoning 124000 (CN); WANG, Zhenghua, Shanghai 201203 (CN); HAO, Jing, Panjin, Liaoning 124000 (CN); YAN, Zhihong, Shanghai 201203 (CN); WANG, Qingbin, Panjin, Liaoning 124000 (CN); XIONG, Yanli, Panjin, Liaoning 124000 (CN); MI, Jun, Panjin, Liaoning 124000 (CN); WANG, Jie, Panjin, Liaoning 124000 (CN); WANG, Haomeng, Shanghai 201203 (CN); GUO, Jun, Panjin, Liaoning 124000 (CN); LIU, Jian, Shanghai 201203 (CN); ZHU, Tao, Shanghai 201203 (CN); QIU, Dongxu, Shanghai 201203 (CN); ZHAO, Xuan, Panjin, Liaoning 124000 (CN); YU, Xuefeng, Shanghai 201203 (CN)
(74) Representative: HGF
(86) International application number: PCT/CN2024/071658
(87) International publication number: WO 2024/149303

(57) **Abstract**

The present invention relates to a steroid-cationic lipid compound as represented by formula (I) and the use thereof. In particular, the prepared LNP can efficiently and stably deliver a bioactive substance to a target cell or organ, and particularly, mRNA LNP prepared therefrom has a relatively good stability and intracellular transfection efficiency, and can cause a relatively high specific antibody response and cellular immune response in an animal.

## Description

### TECHNICAL FIELD

The present disclosure pertains to the field of biopharmaceuticals, and particularly relates to a steroid-cationic lipid compound and use thereof in the delivery of a bioactive substance.

### BACKGROUND

Nucleic acid drugs primarily refer to compounds containing nucleotide or deoxyribonucleotide structures with genetic characteristics and pharmacological activity, which can be used for treating tumors, tissue regeneration, wound healing, pulmonary fibrosis, inflammatory diseases, microbial infections, etc. After being injected into the human body, nucleic acid drugs require a highly efficient and safe drug delivery system to deliver them to the lesion site. This drug delivery system must remain in the body for a sufficient duration to accurately target the lesion site while avoiding damage to normal cells.

The most widely used delivery carriers for nucleic acid drugs at present are lipid nanoparticles and LNP formulations thereof. These carriers have features such as enhancing the efficacy of gene drugs and enabling targeted delivery, protecting nucleic acids from rapid degradation *in vivo,* prolonging circulation time, and improving targeted delivery. Among these, cationic lipid compounds play a critical role in the encapsulation and release of nucleic acids. For example, Patent CN 114773217 B discloses a cationic lipid for delivering nucleic acids, which is described by the following structure: 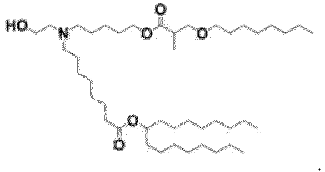 The compound described above is combined with other lipid components in specific ratios to form lipid nanoparticles for delivering nucleic acid drugs. Patent CN 114044741 B discloses a compound with the following structure: 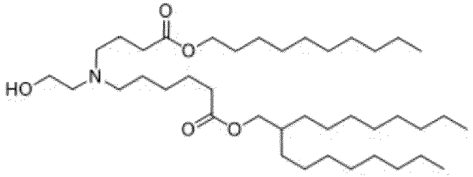 . This compound exhibits high cellular transfection efficiency, low or no cytotoxicity, and enables high and sustained expression in animal bodies for nucleic acid delivery. Patent CN107922364A discloses a lipid and lipid nanoparticle formulation for nucleic acid delivery, with the compound represented by the following Markush structure: 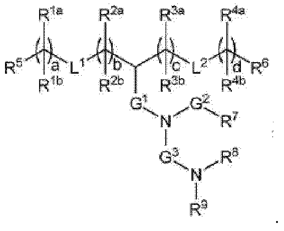 This compound can enhance the activity of nucleic acid drugs and improve the *in vivo* tolerance of LNPs.

It can be seen that cationic lipids for use in nucleic acid drug delivery have been disclosed in the prior art. However, the prior art also reveals that even if cationic lipid compounds are structurally similar, some structurally similar cationic lipid compounds and LNPs thereof still fail to simultaneously achieve high intracellular transfection efficiency, low cytotoxicity, high expression levels, prolonged sustained expression duration, etc. Therefore, further exploration of cationic lipid compounds and LNP delivery systems thereof remains necessary.

### SUMMARY

The present disclosure is based on the following findings: Steroids can influence cellular recognition pathways due to their specific structures, and cationic lipids can fully encapsulate nucleic acid drugs to promote cellular uptake. When combined with nucleic acid drugs, the cationic lipids effectively encapsulate the nucleic acid drugs, thereby enhancing the efficacy, safety, and immunogenicity of nucleic acid drug delivery. By linking a cationic lipid compound with a specific structure to a steroid via a hydrophobic chain, the resulting steroid-cationic lipid simultaneously achieves high intracellular transfection efficiency of nucleic acid drugs, low cytotoxicity, high expression levels, and prolonged sustained expression duration, while demonstrating broad application prospects. In one aspect, the present disclosure provides a cationic lipid compound having a structure represented by formula (I): 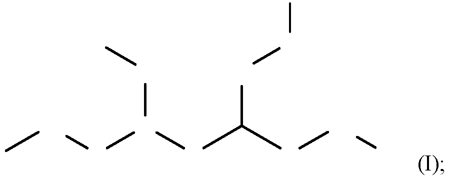
or a stereoisomer thereof, a tautomer thereof, and a pharmaceutically acceptable salt thereof, wherein
L₁, L₂, and L₃ are independently selected from: a chemical bond (-), -O-, -O(C=O)O-, -(C=O)NRa-, -NRa(C=O)-, - NRa-, -O(C=O)-, -(C=O)O-, -C(=O)-, -S(O)x-, -OS(O)xO-, -S-S-, -C(=O)S-, -SC(=O)-, -NRaC(=O)NRa-, - OC(=O)NRa-, -NRaC(=O)O-, -OC(=O)S-, -SC(=O)O-, -P(O)(ORa)O-, and -OP(O)(ORa)O-, wherein x is selected from 0, 1, and 2; Ra is H or C₁-C₁₂ hydrocarbyl;
G₁, G₂, G₃, G₄, and G₅ are each independently selected from: a chemical bond (-), -C₁-C₂₄ alkylene, -C₂-C₂₄ alkenylene, -C₃-C₈ cycloalkylene, and -C₃-C₈ cycloalkenylene;
R₁ is selected from H, -OR₅, -NR₅R₆, -NR₅C(=O)R₆, -C(=O)OR₅, -OC(=O)R₅, -CN, -R₈, and 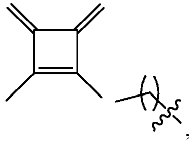 wherein R₅ and R₆ are each independently selected from H, -C₁-C₁₂ alkyl, -C₂-C₁₂ alkenyl, -C₂-C₁₂ alkynyl, -C₁-C₁₂ alkoxy, -C₃-C₈ cycloalkyl, -C₃-C₈ cycloalkenyl, and -C₃-C₈ cycloalkynyl; R₇ is -NR₇'R₇", and R₇' and R₇" are each independently selected from C₁-C₆ alkyl, C₂-C₃ alkenyl, and H; R₈ is selected from pyrazole, imidazole, piperidine, pyrrole, pyrrolidine, piperazine, indole, purine, and morpholine, and the group is optionally substituted with C₁-C₆ alkyl, C₁-C₆ alkenyl, halogen, and hydroxy; n is an integer selected from 1-10 (specifically, n is selected from 1, 2, 3, 4, 5, 6, 7, 8, 9, and 10); and
R₂, R₃, and R₄ are each independently selected from a naturally occurring or non-naturally occurring steroid group, C₆-C₂₄ alkyl, C₆-C₂₄ alkenyl, and C₆-C₂₄ alkynyl,
provided that at least one of R₂, R₃, and R₄ is a naturally occurring or non-naturally occurring steroid group.

In some embodiments, L₁, L₂, and L₃ are each independently selected from: -O(C=O)O-, -O(C=O)-, -(C=O)O-, - C(=O)-, -(C=O)NRₐ-, -NRₐ(C=O)-, -OC(=O)NRₐ-, -NRₐC(=O)O-, -S(O)ₓ₋, -SC(=O)-, and -C(=O)S-, wherein x is selected from 0, 1, and 2; Ra is H or C₁-C₁₂ hydrocarbyl;
G₁, G₂, G₃, G₄, and G₅ are each independently selected from: a chemical bond (-), -C₁₋₁₈ alkylene, -C₂₋₁₈ alkenylene, -C₃₋₈ cycloalkylene, and -C₃₋₈ cycloalkenylene; and
R₁ is selected from H, -OR₅, -NR₅R₆, and -R₈, wherein R₅ and R₆ are each independently selected from H, -C₁-C₁₂ alkyl, -C₂-C₁₂ alkenyl, -C₂-C₁₂ alkynyl, -C₁-C₁₂ alkoxy, -C₃-C₈ cycloalkyl, -C₃-C₈ cycloalkenyl; R₈ is selected from pyrazole, imidazole, pyrrole, pyrrolidine, piperidine, and methylpiperidine.

In some embodiments, L₁, L₂, and L₃ are each independently selected from: -O(C=O)O-, -O(C=O)-, -(C=O)O-, - C(=O)-, -C(=O)S-, -SC(=O)-, -NHC(=O)O-, and -OC(=O)NH-;
G₁, G₂, G₃, G₄, and G₅ are each independently selected from: a chemical bond (-) and -C₁-C₁₀ alkylene; and
R₁ is selected from -OR₅, -NR₅R₆, and -R₈, wherein R₅ and R₆ are each independently selected from H and -C₁-C₅ alkyl; R₈ is selected from pyrazole, imidazole, pyrrole, pyrrolidine, piperidine, and methylpiperidine.

In some embodiments, in the cationic lipid compound of the present disclosure, the steroid is a compound comprising the following carbon skeleton: 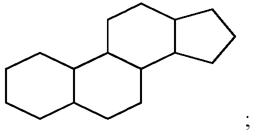 preferably, the steroid is selected from: avenasterol, β-sitosterol, brassicasterol, ergocalciferol, campesterol, cholestanol, cholesterol, coprostanol, dehydrocholesterol, desmosterol, dihydroergocalciferol, dihydrocholesterol, dihydroergosterol, dinosterol, epicholesterol, ergosterol, fucosterol, hexahydrolumisterol, hydroxycholesterol, lanosterol, lumisterol, saringosterol, sitostanol, sitosterol, stigmastanol, stigmasterol, cholic acid, glycocholic acid, taurocholic acid, deoxycholic acid, and/or lithocholic acid.

In some embodiments, the steroid group described herein is a sterol group.

In some embodiments, the sterol described herein is an animal sterol or an oxidized or reduced form thereof; and/or, the sterol is a plant sterol or an oxidized or reduced form thereof; and/or, the sterol is a synthetic sterol or an oxidized or reduced form thereof.

In some embodiments, the sterol described herein is selected from cholesterol, an oxidized form of cholesterol, a reduced form of cholesterol, alkyl lithocholate, stigmasterol, stigmastanol, campesterol, ergosterol, and/or sitosterol.

In some embodiments, the steroid group described herein has the following structure: 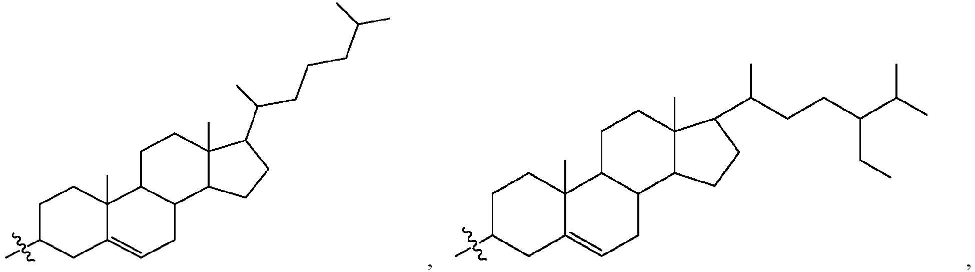 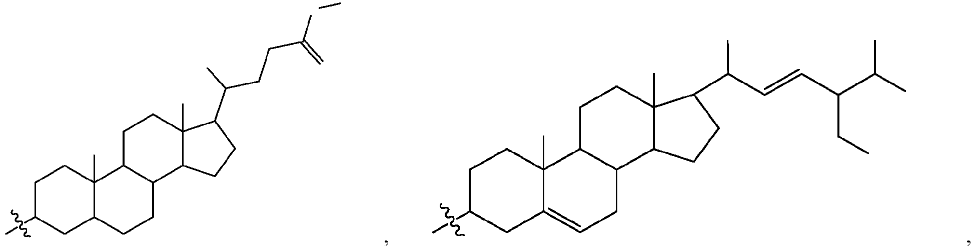 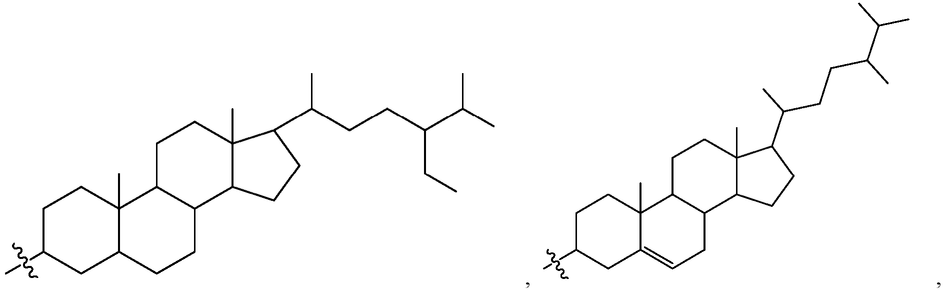 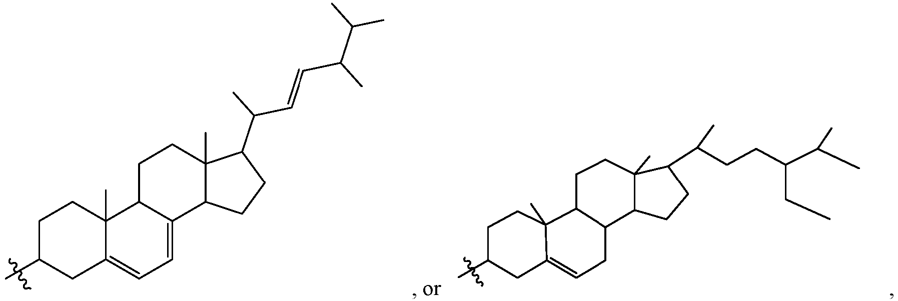 wherein R is C₁₋₂₀ alkyl, including but not limited to, (C1, C2, C3, C4, C5, C6, C7, C8, C9, C10, C11, C12, C13, C14, C15, C16, C17, C18, C19, and C20 alkyl).

In some embodiments, the lipid compound of formula (I) of the present disclosure is a compound of formula (II), 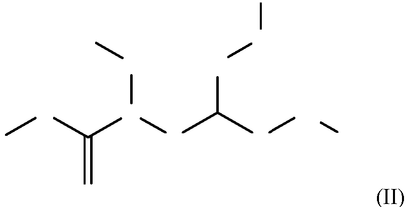 or a stereoisomer thereof, a tautomer thereof, and a pharmaceutically acceptable salt thereof, wherein L₂, L₃, G₁, G₂, G₃, G₄, R₁, R₂, R₃, and R₄ are as defined herein.

In some embodiments, the lipid compound of formula (I) of the present disclosure is a compound of formula (III), 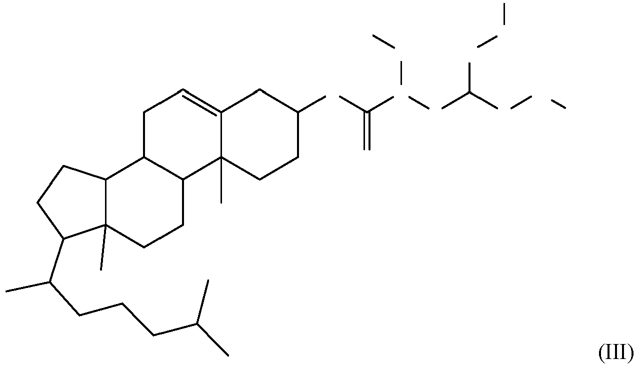 or a stereoisomer thereof, a tautomer thereof, and a pharmaceutically acceptable salt thereof, wherein L₂, L₃, G₁, G₂, G₃, G₄, R₁, R₂, and R₃ are as defined herein.

In some embodiments, the lipid compound of formula (I) of the present disclosure is a compound of formula (IV), 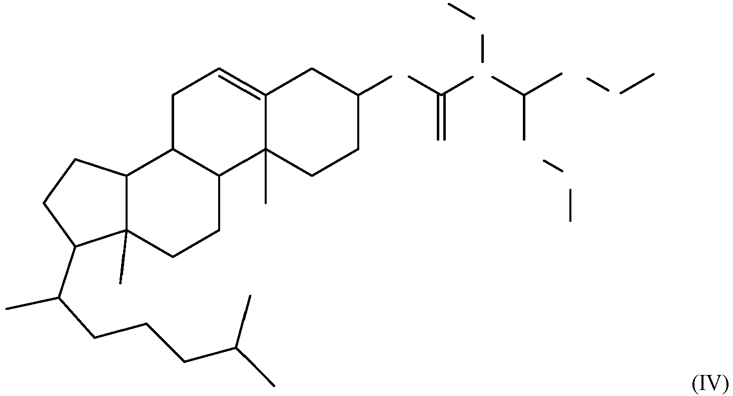 or a stereoisomer thereof, a tautomer thereof, and a pharmaceutically acceptable salt thereof, wherein L₂, L₃, G₂, G₃, G₄, R₁, R₂, and R₃ are as defined herein.

In some embodiments, the lipid compound of formula (I) of the present disclosure is a compound of formula (V), 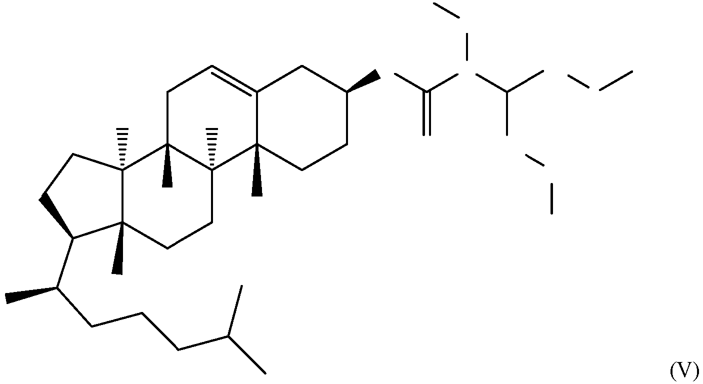 or a stereoisomer thereof, a tautomer thereof, and a pharmaceutically acceptable salt thereof, wherein L₂, L₃, G₂, G₃, G₄, R₁, R₂, and R₃ are as defined herein.

In some embodiments, in the cationic lipid compound of the present disclosure, L₂ and L₃ are each independently selected from: -O(C=O)O-, -O(C=O)-, -(C=O)O-, -C(=O)-, -C(=O)S-, -SC(=O)-, -NHC(=O)O-, and -OC(=O)NH-; G₂, G₃, and G₄ are each independently selected from -C₁-C₁₀ alkylene;
R₁ is selected from -OR₅, -NR₅R₆, and -R₈, wherein R₅ and R₆ are each independently selected from H and -C₁-C₅ alkyl; R₈ is selected from pyrazole, imidazole, pyrrole, pyrrolidine, piperidine, and methylpiperidine;
R₂ and R₃ are each independently selected from: 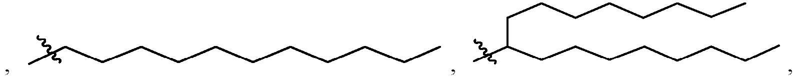 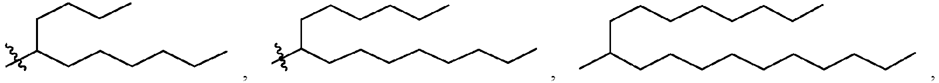 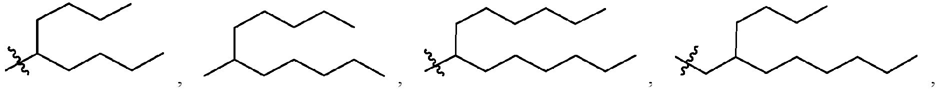 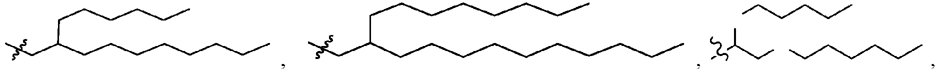 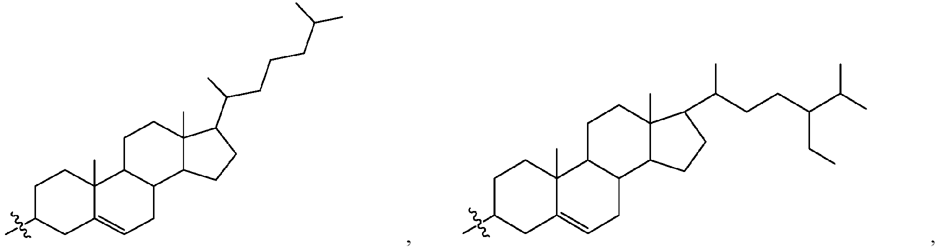 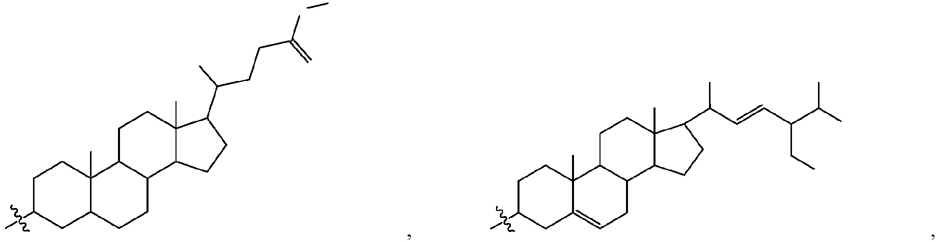 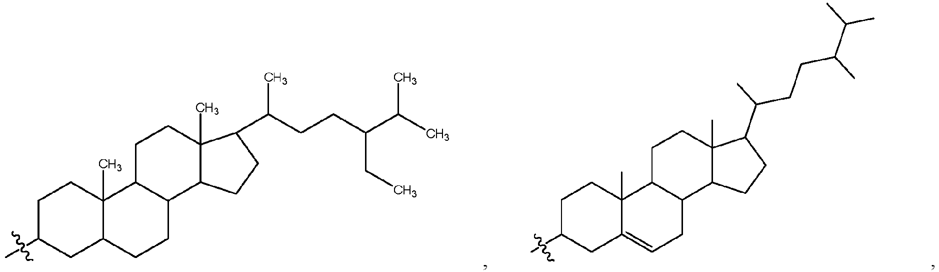 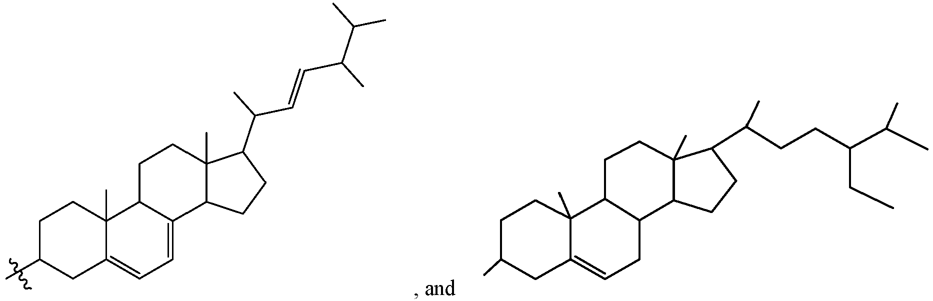 ; and
preferably, R₂ and R₃ are each independently selected from: 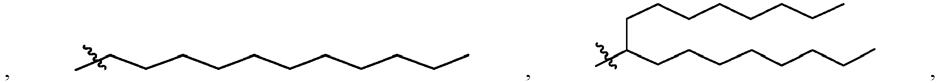 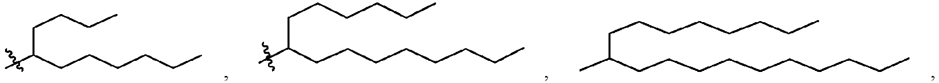 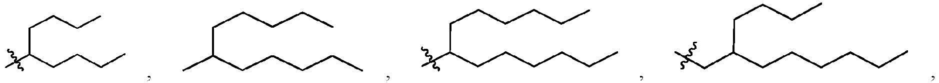 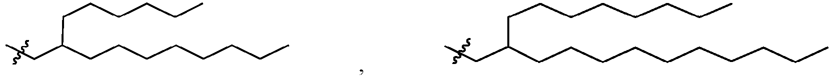 , and

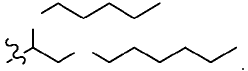

In some embodiments, the cationic lipid compound of the present disclosure is selected from: 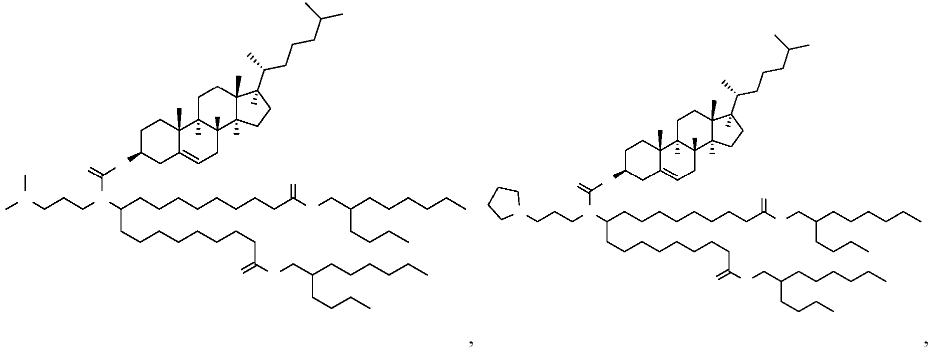 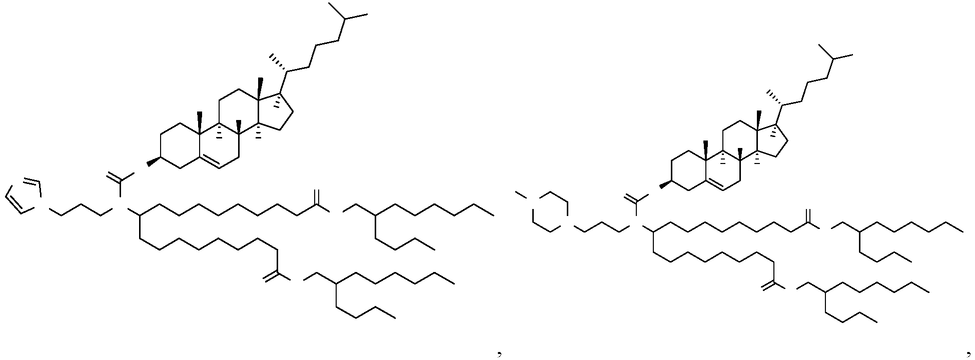 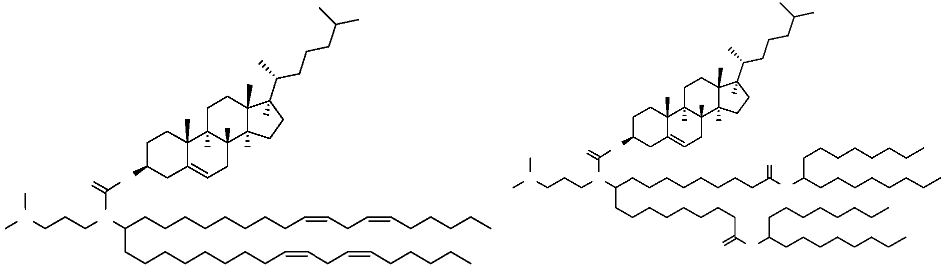 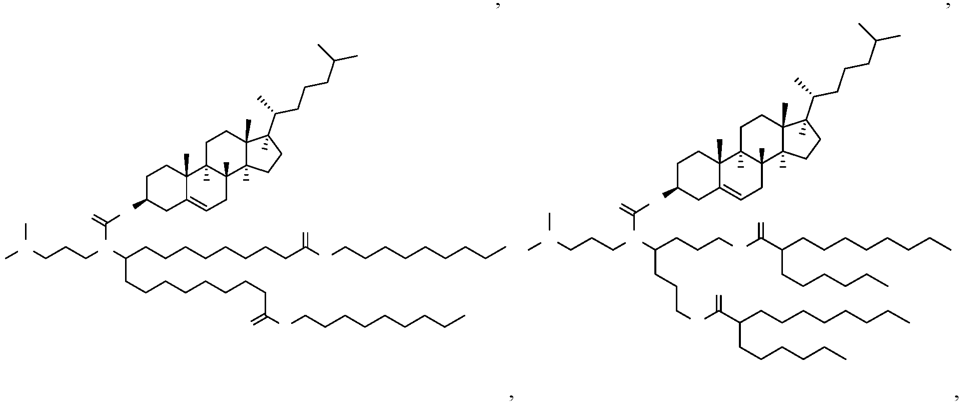 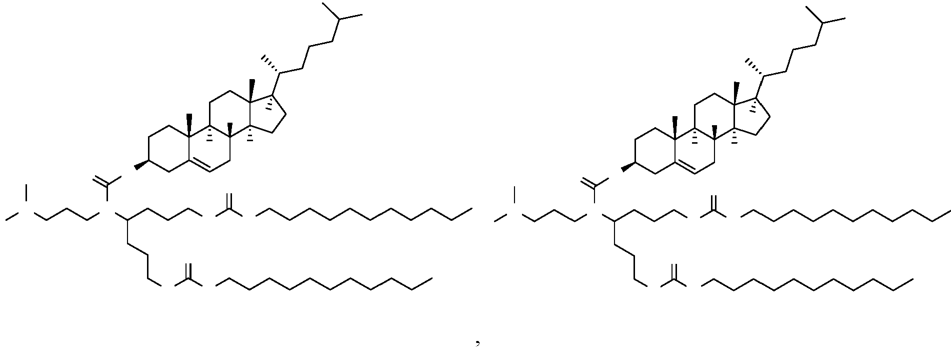 , and 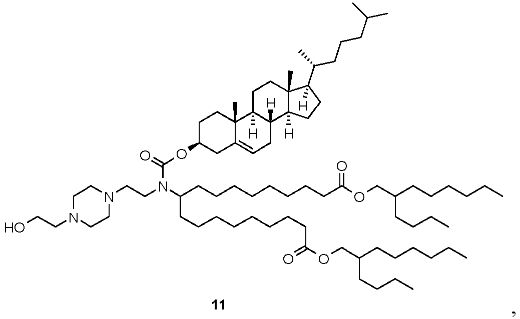 or a stereoisomer thereof, a tautomer thereof, and a pharmaceutically acceptable salt thereof.

In some embodiments, the present disclosure provides an LNP comprising the cationic lipid compound described in the present disclosure.

In some embodiments, the LNP described in the present disclosure further comprises a polyethylene glycol lipid and at least one second lipid, wherein the second lipid is selected from: a neutral lipid, a zwitterionic lipid, and an anionic lipid.

In some embodiments, the polyethylene glycol lipid is selected from: 2-[(polyethylene glycol)-2000]-N,N-ditetradecylacetamide (ALC-0159), 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-disterol glycerol (PEG-DSG), PEG-dipalmitoyl, PEG-dioleyl, PEG-distearyl, PEG-diacylglycerol amide (PEG-DAG), PEG-dipalmitoyl phosphatidylethanolamine (PEG-DPPE), and PEG-1,2-dimyristoyloxypropyl-3-amine (PEG-c-DMA);
and/or,
the neutral lipid is selected from: 1,2-distearoyl-sn-glycero-3-phosphocholine (DSPC), 1,2-dipalmitoyl-sn-glycero-3-phosphocholine (DPPC), 1,2-dioleoyl-sn-glycero-3-phosphoethanolamine (DOPE), 1,2-dipalmitoyl-sn-glycero-3-phosphoethanolamine (DPPE), 1,2-dimyristoyl-sn-glycero-3-phosphoethanolamine (DMPE), 2-dioleoyl-sn-glycero-3-phospho-(1'-rac-glycerol) (DOPG), oleoyl phosphatidylcholine (POPC), and 1-palmitoyl-2-oleoyl phosphatidylethanolamine (POPE).

In some embodiments, in the LNP, a molar ratio of the cationic lipid:the second lipid:the PEG-lipid described in the present disclosure is 5-100:0.5-80:1-50; preferably, the molar ratio of the cationic lipid:the second lipid:the PEG-lipid described in the present disclosure is 40-60:40-60:1-10; and more preferably, the molar ratio of the cationic lipid:the second lipid:the PEG-lipid described in the present disclosure is 49.25:49.25:1.5.

In some embodiments, a nitrogen-to-phosphorus ratio of the LNP is in the range of 1:1 to 15:1; for example, the nitrogen-to-phosphorus ratio may be 1:1, 1.5:1, 2:1, 2.5:1, 3:1, 3.5:1, 4:1, 4.5:1, 5:1, 5.5:1, 6:1, 6.5:1, 7:1, 7.5:1, 8:1, 8.5:1, 9:1, 9.5:1, 10:1, 10.5:1, 11:1, 11.5:1, 12:1, 12.5:1, 13:1, 13.5:1, 14:1, 14.5:1, or 15:1; preferably, the nitrogen-to-phosphorus ratio of the LNP is in the range of 2:1 to 10:1; more preferably, the nitrogen-to-phosphorus ratio of the LNP is in the range of 4:1 to 8:1; and most preferably, the nitrogen-to-phosphorus ratio of the LNP is in the range of 6:1.

In some embodiments, the LNP has a diameter of 15 nm to 300 nm, including but not limited to, 15 nm, 20 nm, 25 nm, 30 nm, 35 nm, 40 nm, 45 nm, 50 nm, 55 nm, 60 nm, 65 nm, 70 nm, 80 nm, 100 nm, 150 nm, 200 nm, 250 nm, or 300 nm. Preferably, the LNP has a diameter of 50 nm to 100 nm.

In some embodiments, the present disclosure further provides use of the cationic lipid compound described in the present disclosure and the LNP prepared therefrom in preparing a bioactive substance delivery system.

In some embodiments, the bioactive substance described herein may be a small-molecule compound, a nucleic acid, an oligopeptide, etc.; preferably, the bioactive substance is a nucleic acid.

In some embodiments, the bioactive substance described herein is a DNA or an RNA.

In some embodiments, the DNA described herein includes a non-coding DNA (antisense DNA) or a coding DNA.

In some embodiments, the RNA described herein includes an antisense RNA, an saRNA, an mRNA, an lncRNA, an miRNA, an siRNA, a piRNA, a gRNA, a tsRNA, a circRNA, and a self-replicating mRNA.

In some embodiments, the nucleic acid is used for preventing and/or treating cancer, inflammation, fibrotic disease, autoimmune disease, infection, psychiatric disorder, hematological disorder, chromosomal disease, genetic disease, connective tissue disease, digestive disease, ear-nose-throat disease, endocrine disease, eye disease, reproductive disease, heart disease, kidney disease, lung disease, metabolic disorder, oral disease, musculoskeletal disease, neonatal screening, nutritional disease, parasitic disease, skin disease, etc.

In some embodiments, the bioactive substance delivery system is an mRNA vaccine.

In some embodiments, the mRNA vaccine may be used for preventing cancer, a viral infection, a bacterial infection, a fungal infection, etc. The virus includes, but is not limited to: norovirus, Ebola virus, coronavirus (including novel coronavirus SARS-CoV-2), cytomegalovirus, Dengue virus, Zika virus, coxsackievirus, enterovirus, hepatitis virus, herpes simplex virus, human papilloma virus, influenza virus, Marburg virus, measles virus, poliovirus, rabies virus, rotavirus, measles virus, etc.

In some embodiments, the present disclosure further provides use of the LNP for preparing an mRNA vaccine.

The preparation method for the LNP of the present disclosure may employ conventional methods in the art, such as the microfluidic technology.

In some embodiments, the present disclosure further provides a medicament comprising the bioactive substance and the LNP described herein.

In some embodiments, the medicament further comprises a pharmaceutically acceptable auxiliary material.

In some embodiments, the pharmaceutically acceptable auxiliary material described in the present disclosure is, for example, a carrier, an adjuvant, or a diluent.

In some embodiments, the steroid-cationic lipid compound, the LNP, or the medicament described in the present disclosure may be used to deliver the bioactive substance via oral administration, inhalation, or injection.

In some embodiments, the medicament described in the present disclosure is a gene drug.

In some embodiments, the present disclosure further provides a method for delivering a bioactive substance, which comprises administering to a human in need thereof the medicament described in the present disclosure.

The present disclosure has the following beneficial effects:
The steroid-cationic lipid compound described in the present disclosure, when used as an mRNA LNP, exhibits enhanced stability and transfection efficiency, and induces relatively high specific antibody response and cellular immune response *in vivo.*

### BRIEF DESCRIPTION OF THE DRAWINGS

FIG. 1 shows the transfection efficiency of Luc-mRNA-LNP in HEK293T cells as measured by bioluminescence.
FIG. 2a shows the serum antigen-specific antibody titer in mice immunized with SARS-CoV-2 antigen mRNA-LNP, as measured by ELISA.
FIG. 2b shows the IFN-γ+ cellular response level in PBMCs of mice immunized with SARS-CoV-2 antigen mRNA-LNP, as measured by ELSPOT.

### DETAILED DESCRIPTION

The technical solutions in the examples of the present disclosure will be described clearly and completely below with reference to the drawings. It is obvious that the described examples are only a part of the examples of the present disclosure, rather than all of them. Based on the examples of the present disclosure, all other examples obtained by those of ordinary skills in the art without creative work shall fall within the protection scope of the present disclosure.

The term "LNP" (lipid nanoparticle) in the present disclosure refers to a particle with a nanoscale size (e.g., 1 nm to 1000 nm) comprising one or more types of lipid molecules.

The term "gene drug" in the present disclosure generally refers to a drug composed of a vector or a delivery system containing an engineered genetic construct. The active ingredient may be a DNA, an RNA, a genetically modified virus, a bacterium, or a cell, which introduces an exogenous gene into a target cell or tissue to replace, compensate for, block, or correct a specific gene for therapeutic or prophylactic purposes.

The term "nucleic acid" of the present disclosure refers to a polymer containing at least two kinds of deoxyribonucleotides or ribonucleotides in either single- or double-stranded form and includes a DNA, an RNA, and a hybrid thereof.

The term "lipid" of the present disclosure refers to a group of organic compounds that include, but are not limited to, esters of fatty acids and are generally characterized by being poorly soluble in water, but soluble in many organic solvents.

The term "cationic lipid" of the present disclosure refers to a lipid molecule capable of being positively charged.

The term "neutral lipid" of the present disclosure refers to an uncharged non-phosphoglyceride lipid molecule.

The term "polyethylene glycol lipid" of the present disclosure refers to a molecule comprising a lipid portion and a polyethylene glycol portion.

The term "delivery system" of the present disclosure refers to a formulation or composition that regulates the spatial, temporal, and dose distribution of a bioactive ingredient in an organism.

### Example 1: Synthesis of Compound 1

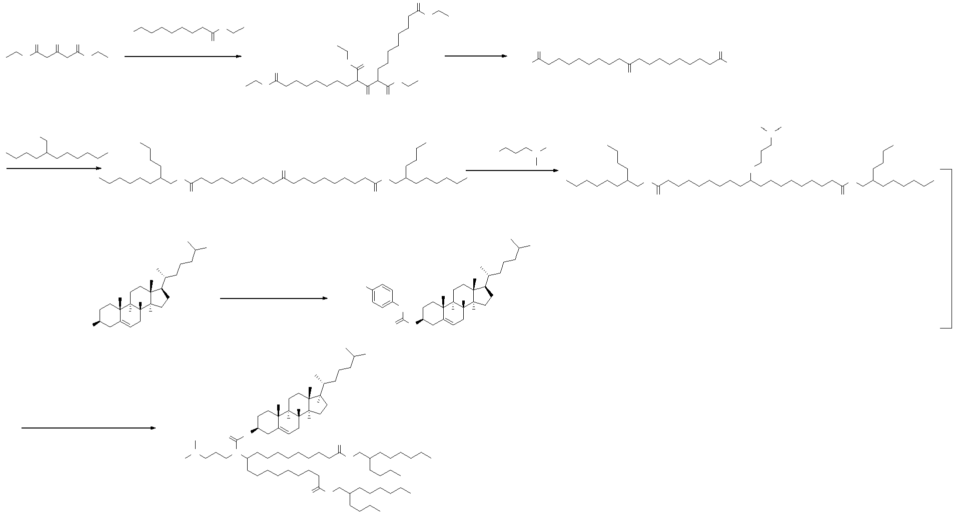

### Step 1: Synthesis of compound 1a

Diethyl 1,3-acetonedicarboxylate (21.15 g, 104.5 mmol) was dissolved in 50 mL of ethanol. Sodium ethoxide (7.11 g, 104.5 mmol) was added, and the mixture was heated at reflux. Ethyl 8-bromooctanoate (7.11 g, 104.5 mmol) was slowly added dropwise. After the mixture was reacted under reflux for 2 h, ethanol (50 mL) and sodium ethoxide (7.11 g, 104.5 mmol) were added, and the mixture was heated at reflux. Ethyl 8-bromooctanoate (7.11 g, 104.5 mmol) was slowly added dropwise. The mixture was reacted under reflux overnight. The reaction solution was concentrated to dryness, and methyl tert-butyl ether (150 mL) was added. The mixture was washed once with a saturated aqueous ammonium chloride solution and once with purified water. The organic phase was dried over anhydrous sodium sulfate and concentrated to give compound 1a, which was directly used in the next step.

### Step 2: Synthesis of compound 1b

50 mL of acetic acid and 120 mL of concentrated hydrochloric acid were added to compound 1a obtained above. The mixture was heated to reflux and reacted for 18 h. After completion of the reaction, the reaction solution was concentrated, washed once with purified water, and filtered. The filter cake was added to dichloromethane for crystallization, filtered, and dried to give compound 1b (8.5 g, white solid, yield: 24%).

### Step 3: Synthesis of compound 1c

Compound 1b (8.0 g, 23.39 mnol) was dissolved in dichloromethane (80 mL). 2-Butyl-1-n-octanol (8.7 g, 46.78 mmol), EDC·HCl (6.7 g, 34.93 mmol), and DMAP (1.14 g, 9.59 mmol) were added. The mixture was stirred at room temperature for 16-20 h, and the reaction was completed as detected by HPLC. The reaction solution was concentrated at 40 °C until no liquid was distilled out. n-Hexane was added to dissolve the residue, and the mixture was washed twice with acetonitrile. The n-hexane phase was taken and concentrated until no liquid was distilled out. The n-hexane phase was separated by column chromatography to give compound 1c (5.0 g, pale yellow oil, yield: 32%).

### Step 4: Synthesis of compound 1d

Compound 1c (5.0 g, 7.36 mmol) was dissolved in ethanol (50 mL), and a 2-methylpyridine borane complex (1.57 g, 14.72 mmol), N,N-dimethyl-1,3-propanediamine (0.75 g, 7.36 mmol), and acetic acid (0.5 g) were added. The mixture was reacted at room temperature for 24 h. After completion of the reaction, the reaction solution was concentrated until no liquid was distilled out. Ethyl acetate was added, and after achieving complete dissolution, the mixture was washed once with a 10% aqueous hydrochloric acid solution, once with a saturated aqueous sodium sulfite solution, and once with purified water. The organic phase was dried over anhydrous sodium sulfate and concentrated. The obtained oil was dissolved in a methanol:isopropanol (1:4) mixed solution. 5% palladium on carbon (1 g) was added, and the mixture was stirred at room temperature for 8 h. The resulting mixture was filtered through celite, and the filtrate was concentrated and separated by column chromatography to give compound 1d (3.0 g, pale yellow oil, yield: 53%).

### Step 5: Synthesis of compound 1e

Cholesterol (10.0 g, 25.86 mmol) was dissolved in 100 mL of dichloromethane. DMAP (0.6 g, 5.17 mmol) and triethylamine (5.23 g, 51.71 mmol) were added. Subsequently, p-nitrophenyl chloroformate (5.7 g, 28.44 mmol) was added in batches, and the reaction mixture was stirred at room temperature for 16 h. The reaction was completed as shown by the TCL plate. The yellow-green reaction solution was added dropwise to acetonitrile, resulting in solid precipitation and crystallization. The mixture was filtered under vacuum, and the filter cake was dried to give compound 1e (13.0 g, white solid, yield: 91%).

### Step 6: Synthesis of compound 1

Compound 1e (13.0 g) was dissolved in dichloromethane. Compound 1d (3.0 g, 3.92 mmol) and DMAP (0.6 g, 5.17 mmol) were added. The mixture was stirred at room temperature for 16-20 h, and the reaction was completed as detected by CAD. The reaction solution was concentrated at 40 °C until no liquid was distilled out. n-Hexane was added to dissolve the residue, and the mixture was washed twice with acetonitrile under stirring. The n-hexane phase was concentrated to dryness and separated by column chromatography to give compound 1 (1.20 g, pale yellow oil, yield: 26%).
MS m/z (ESI): 1178.8 [M+1].
1H NMR (300MHz, CDCl₃): δ 5.45 (t, 1H, J = 5.4 Hz), 4.53-4.46 (m, 1H), 3.98 (d, 4H, J = 5.7 Hz), 3.21-3.10 (m, 2H), 2.54-2.50 (m, 1H), 2.47-2.26 (m, 13H), 2.08-0.88 (m, 115H), 0.70 (s, 3H)

### Example 2: Synthesis of Compound 2

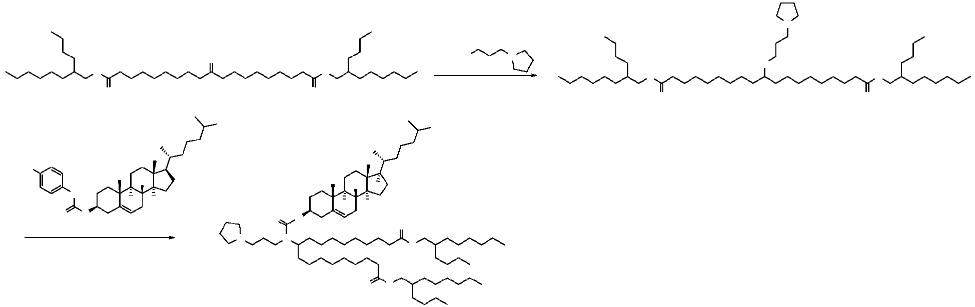

### Step 1: Synthesis of compound 2a

Compound 1c (5.0 g, 7.36 mmol) was dissolved in ethanol (50 mL), and a 2-methylpyridine borane complex (1.57 g, 14.72 mmol), 1-(3-aminopropyl)pyrrolidine (0.94 g, 7.36 mmol), and acetic acid (0.5 g) were added. The mixture was reacted at room temperature for 24 h. After completion of the reaction, the reaction solution was concentrated until no liquid was distilled out. Ethyl acetate was added, and after achieving complete dissolution, the mixture was washed once with a 10% aqueous hydrochloric acid solution, once with a saturated aqueous sodium sulfite solution, and once with purified water. The organic phase was dried over anhydrous sodium sulfate and concentrated. The obtained oil was dissolved in a methanol:isopropanol (1:4) mixed solution. 5% palladium on carbon (1 g) was added, and the mixture was stirred at room temperature for 8 h. The resulting mixture was filtered through celite, and the filtrate was concentrated and separated by column chromatography to give compound 2a (3.2 g, pale yellow oil, yield: 55%).

### Step 2: Synthesis of compound 2

Compound 1e (13.0 g) was dissolved in dichloromethane. Compound 2a (3.2 g, 4.04 mmol) and DMAP (0.6 g, 5.17 mmol) were added. The mixture was stirred at room temperature for 16-20 h, and the reaction was completed as detected by CAD. The reaction solution was concentrated at 40 °C until no liquid was distilled out. n-Hexane was added to dissolve the residue, and the mixture was washed twice with acetonitrile under stirring. The n-hexane phase was concentrated to dryness and separated by column chromatography to give compound 2 (1.12 g, pale yellow oil, yield: 23%).
MS m/z (ESI): 1204.1 [M+1].
1H NMR (300MHz, CDCl₃): δ 5.45 (t, 1H, J = 5.4 Hz), 4.53-4.46 (m, 1H), 3.98 (d, 4H, J = 5.7 Hz), 3.21-3.10 (m, 2H), 2.54-2.52 (m, 1H), 2.51 (t, 4H, J = 5.7 Hz), 2.47-2.26 (m, 13H), 2.08-0.88 (m, 113H), 0.70 (s, 3H)

### Example 3: Synthesis of Compound 3

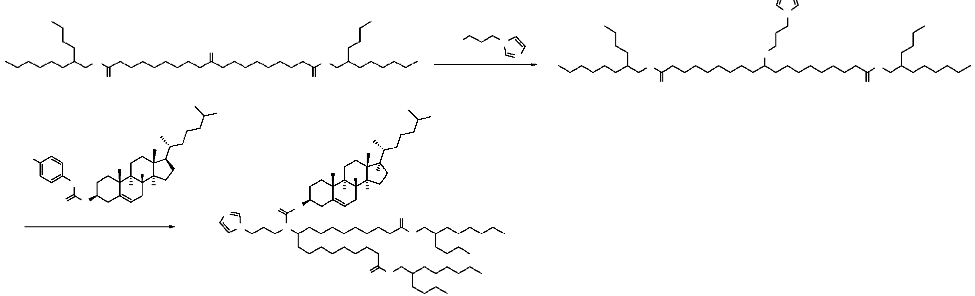

### Step 1: Synthesis of compound 3a

Compound 1c (5.0 g, 7.36 mmol) was dissolved in ethanol (50 mL), and a 2-methylpyridine borane complex (1.57 g, 14.72 mmol), 1-(3-aminopropyl)imidazole (0.92 g, 7.36 mmol), and acetic acid (0.5 g) were added. The mixture was reacted at room temperature for 24 h. After completion of the reaction, the reaction solution was concentrated until no liquid was distilled out. Ethyl acetate was added, and after achieving complete dissolution, the mixture was washed once with a 10% aqueous hydrochloric acid solution, once with a saturated aqueous sodium sulfite solution, and once with purified water. The organic phase was dried over anhydrous sodium sulfate and concentrated. The obtained oil was dissolved in a methanol:isopropanol (1:4) mixed solution. 5% palladium on carbon (1 g) was added, and the mixture was stirred at room temperature for 8 h. The resulting mixture was filtered through celite, and the filtrate was concentrated and separated by column chromatography to give compound 3a (2.9 g, pale yellow oil, yield: 50%).

### Step 2: Synthesis of compound 3

Compound 1e (13.0 g) was dissolved in dichloromethane. Compound 3a (3.2 g, 4.04 mmol) and DMAP (0.6 g, 5.17 mmol) were added. The mixture was stirred at room temperature for 16-20 h, and the reaction was completed as detected by CAD. The reaction solution was concentrated at 40 °C until no liquid was distilled out. n-Hexane was added to dissolve the residue, and the mixture was washed twice with acetonitrile under stirring. The n-hexane phase was concentrated to dryness and separated by column chromatography to give compound 3 (1.16 g, pale yellow oil, yield: 24%).
MS m/z (ESI): 1201.1 [M+1].
1H NMR (300MHz, CDCl₃): δ 7.95 (s, 1H), 7.19 (d, 1H, J = 5.7 Hz), 6.80 (d, 1H, J = 5.7 Hz), 5.45 (t, 1H, J = 5.4 Hz), 4.53-4.46 (m, 1H), 3.98 (d, 4H, J = 5.7 Hz), 3.89 (t, 2H, J = 5.4 Hz), 3.21-3.10 (m, 2H), 2.54-2.52 (m, 1H), 2.51-2.48 (m, 2H), 2.47-2.26 (m, 8H), 2.08-0.88 (m, 110H), 0.70 (s, 3H)

### Example 4: Synthesis of Compound 4

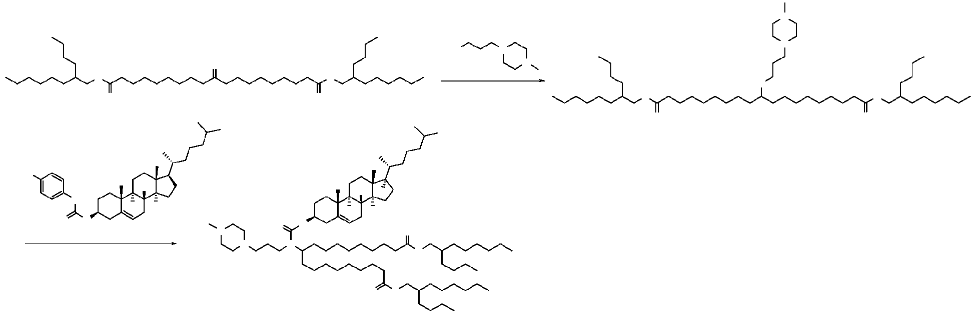

### Step 1: Synthesis of compound 4a

Compound 1c (5.0 g, 7.36 mmol) was dissolved in ethanol (50 mL), and a 2-methylpyridine borane complex (1.57 g, 14.72 mmol), 1-(3-aminopropyl)-4-methylpiperazine (1.16 g, 7.36 mmol), and acetic acid (0.5 g) were added. The mixture was reacted at room temperature for 24 h. After completion of the reaction, the reaction solution was concentrated until no liquid was distilled out. Ethyl acetate was added, and after achieving complete dissolution, the mixture was washed once with a 10% aqueous hydrochloric acid solution, once with a saturated aqueous sodium sulfite solution, and once with purified water. The organic phase was dried over anhydrous sodium sulfate and concentrated. The obtained oil was dissolved in a methanol:isopropanol (1:4) mixed solution. 5% palladium on carbon (1 g) was added, and the mixture was stirred at room temperature for 8 h. The resulting mixture was filtered through celite, and the filtrate was concentrated and separated by column chromatography to give compound 4a (2.9 g, pale yellow oil, yield: 48%).

### Step 2: Synthesis of compound 4

Compound 1e (13.0 g) was dissolved in dichloromethane. Compound 4a (3.3 g, 4.04 mmol) and DMAP (0.6 g, 5.17 mmol) were added. The mixture was stirred at room temperature for 16-20 h, and the reaction was completed as detected by CAD. The reaction solution was concentrated at 40 °C until no liquid was distilled out. n-Hexane was added to dissolve the residue, and the mixture was washed twice with acetonitrile under stirring. The n-hexane phase was concentrated to dryness and separated by column chromatography to give compound 4 (1.54 g, pale yellow oil, yield: 31%).
MS m/z (ESI): 1233.1 [M+1].
1H NMR (300MHz, CDCl₃): δ 5.45 (t, 1H, J = 5.4 Hz), 4.53-4.46 (m, 1H), 3.98 (d, 4H, J = 5.7 Hz), 3.21-3.10 (m, 2H), 2.34-2.12 (m, 25H), 2.08-0.88 (m, 109H), 0.70 (s, 3H)

### Example 5: Synthesis of Compound 5

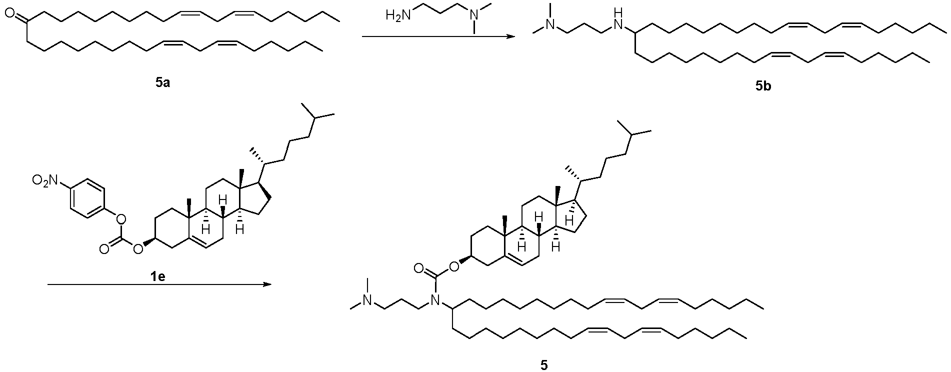

### Step 1: Synthesis of compound 5b

Compound 5a (3.16 g, 6.0 mmol) and 3-dimethylamino-1-propylamine (612 mg, 6.0 mmol) were dissolved in dichloromethane (25 mL), and sodium triacetoxyborohydride (1.80 g, 8.49 mmol) and AcOH (0.36 g, 6.0 mmol) were added. The mixture was stirred at room temperature for 2 days. After completion of the reaction, the reaction was quenched with 1 N NaOH, and the product was extracted with a mixture of hexane and ethyl acetate. The organic extract was successively washed with water/brine (1:1) and brine, dried over anhydrous sodium sulfate, and concentrated to give compound 5b (3.55 g, yellow oil).

### Step 2: Synthesis of compound 5

Compound 1e (13.0 g) was dissolved in dichloromethane. Compound 5b (2.5 g, 4.04 mmol) and DMAP (0.6 g, 5.17 mmol) were added. The mixture was stirred at room temperature for 16-20 h, and the reaction was completed as detected by CAD. The reaction solution was concentrated at 40 °C until no liquid was distilled out. n-Hexane was added to dissolve the residue, and the mixture was washed twice with acetonitrile under stirring. The n-hexane phase was concentrated to dryness and separated by column chromatography to give compound 5 (1.66 g, pale yellow oil, yield: 40%).
MS m/z (ESI): 1026.0 [M+1].
1H NMR (300MHz, CDCl₃): δ 5.45-5.43 (m, 5H), 5.38-5.33 (m, 4H), 4.53-4.48 (m, 4H), 3.41-3.36 (m, 1H), 2.90-2.64 (m, 6H), 2.47-2.16 (m, 22H), 2.08-0.88 (m, 79H), 0.70 (s, 3H)

### Example 6: Synthesis of Compound 6

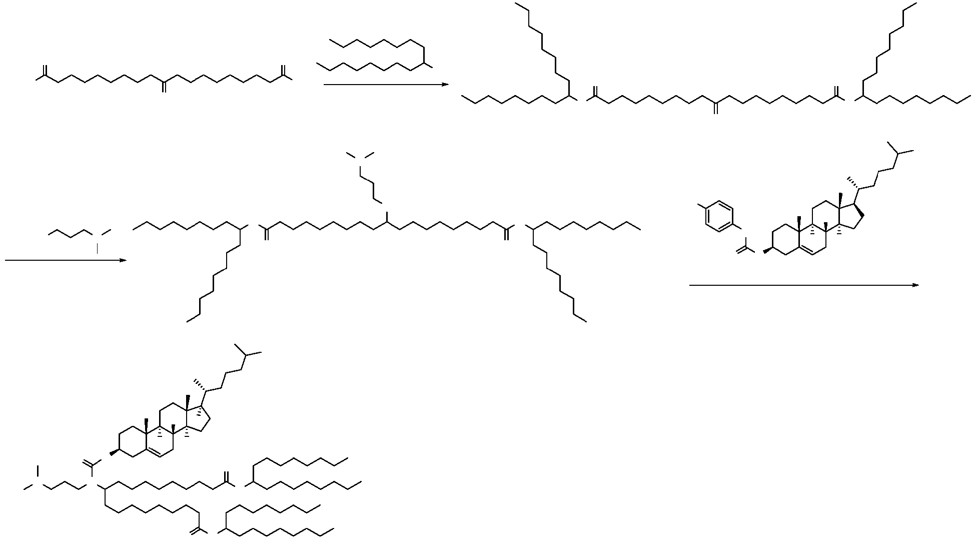

### Step 1: Synthesis of compound 6a

Compound 1b (3.4 g, 10 mmol) was dissolved in dichloromethane (20 mL). 9-Aminoheptadecane (5.1 g, 20 mmol), EDC·HCl (2.9 g, 15 mmol), and DMAP (0.5 g, 4 mmol) were added. The mixture was stirred at room temperature for 16-20 h, and the reaction was completed as detected by HPLC. The reaction solution was concentrated at 40 °C until no liquid was distilled out. n-Hexane was added to dissolve the residue, and the mixture was washed twice with acetonitrile. The n-hexane phase was taken and concentrated until no liquid was distilled out. The n-hexane phase was separated by column chromatography to give compound 6a (3.3 g, pale yellow oil, yield: 41%).

### Step 2: Synthesis of compound 6b

Compound 6a (3.3 g, 4 mmol) was dissolved in ethanol (30 mL), and a 2-methylpyridine borane complex (0.97 g, 8 mmol), N,N-dimethyl-1,3-propanediamine (0.41 g, 4 mmol), and acetic acid (0.3 g) were added. The mixture was reacted at room temperature for 24 h. After completion of the reaction, the reaction solution was concentrated until no liquid was distilled out. Ethyl acetate was added, and after achieving complete dissolution, the mixture was washed once with a 10% aqueous hydrochloric acid solution, once with a saturated aqueous sodium sulfite solution, and once with purified water. The organic phase was dried over anhydrous sodium sulfate and concentrated. The obtained oil was dissolved in a methanol:isopropanol (1:4) mixed solution. 5% palladium on carbon (0.5 g) was added, and the mixture was stirred at room temperature for 8 h. The resulting mixture was filtered through celite, and the filtrate was concentrated and separated by column chromatography to give compound 6b (2.0 g, pale yellow oil, yield: 55%).

### Step 3: Synthesis of compound 6

Compound 1e (8.0 g) was dissolved in dichloromethane. Compound 6b (2.0 g, 2.2 mmol) and DMAP (0.4 g, 3.3 mmol) were added. The mixture was stirred at room temperature for 16-20 h, and the reaction was completed as detected by CAD. The reaction solution was concentrated at 40 °C until no liquid was distilled out. n-Hexane was added to dissolve the residue, and the mixture was washed twice with acetonitrile under stirring. The n-hexane phase was concentrated to dryness and separated by column chromatography to give compound 6 (0.90 g, pale yellow oil, yield: 31%).
MS m/z (ESI): 1316.3 [M+1].
1H NMR (300MHz, CDCl₃): δ 8.16 (s, 2H), 5.45 (t, 1H, J = 5.4 Hz), 4.53-4.46 (m, 1H), 3.41-3.30 (m, 3H), 2.54-2.26 (m, 14H), 2.08-0.88 (m, 138H), 0.70 (s, 3H)

### Example 7: Synthesis of Compound 7

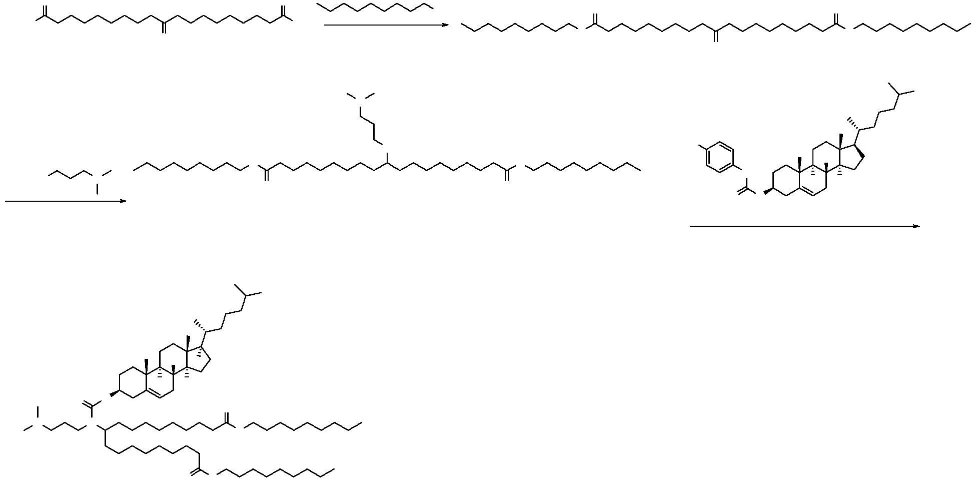

### Step 1: Synthesis of compound 7a

Compound 1b (3.4 g, 10 mmol) was dissolved in dichloromethane (20 mL). Nonanethiol (3.2 g, 20 mmol), EDC·HCl (2.9 g, 15 mmol), and DMAP (0.5 g, 4 mmol) were added. The mixture was stirred at room temperature for 16-20 h, and the reaction was completed as detected by HPLC. The reaction solution was concentrated at 40 °C until no liquid was distilled out. n-Hexane was added to dissolve the residue, and the mixture was washed twice with acetonitrile. The n-hexane phase was taken and concentrated until no liquid was distilled out. The n-hexane phase was separated by column chromatography to give compound 7a (2.8 g, pale yellow oil, yield: 45%).

### Step 2: Synthesis of compound 7b

Compound 7a (2.5 g, 4 mmol) was dissolved in ethanol (30 mL), and a 2-methylpyridine borane complex (0.97 g, 8 mmol), N,N-dimethyl-1,3-propanediamine (0.41 g, 4 mmol), and acetic acid (0.3 g) were added. The mixture was reacted at room temperature for 24 h. After completion of the reaction, the reaction solution was concentrated until no liquid was distilled out. Ethyl acetate was added, and after achieving complete dissolution, the mixture was washed once with a 10% aqueous hydrochloric acid solution, once with a saturated aqueous sodium sulfite solution, and once with purified water. The organic phase was dried over anhydrous sodium sulfate and concentrated. The obtained oil was dissolved in a methanol:isopropanol (1:4) mixed solution. 5% palladium on carbon (0.5 g) was added, and the mixture was stirred at room temperature for 8 h. The resulting mixture was filtered through celite, and the filtrate was concentrated and separated by column chromatography to give compound 7b (1.5 g, pale yellow oil, yield: 53%).

### Step 3: Synthesis of compound 7

Compound 1e (8.0 g) was dissolved in dichloromethane. Compound 7b (1.5 g, 2.2 mmol) and DMAP (0.4 g, 3.3 mmol) were added. The mixture was stirred at room temperature for 16-20 h, and the reaction was completed as detected by CAD. The reaction solution was concentrated at 40 °C until no liquid was distilled out. n-Hexane was added to dissolve the residue, and the mixture was washed twice with acetonitrile under stirring. The n-hexane phase was concentrated to dryness and separated by column chromatography to give compound 7 (0.89 g, pale yellow oil, yield: 36%).
MS m/z (ESI): 1125.9 [M+1].
1H NMR (300MHz, CDCl₃): δ 5.45 (t, 1H, J = 5.4 Hz), 4.53-4.46 (m, 1H), 3.41-3.30 (m, 1H), 3.25 (t, 4H, J = 5.4 Hz), 3.21-3.10 (m, 2H), 2.47-2.26 (m, 13H), 2.08-0.88 (m, 103H), 0.70 (s, 3H)

### Example 8: Synthesis of Compound 8

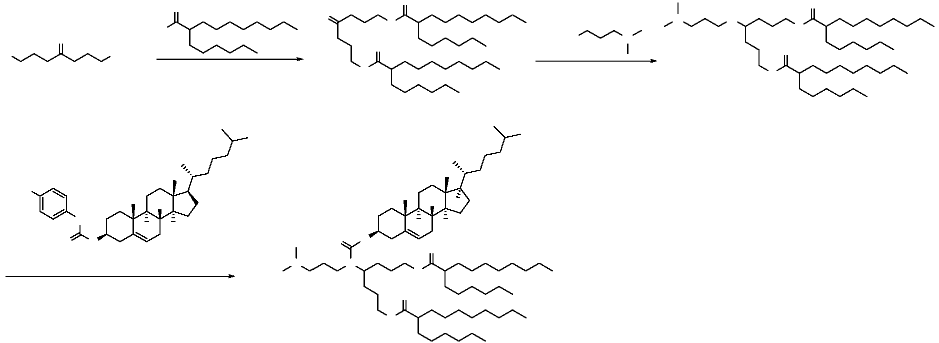

### Step 1: Synthesis of compound 8b

2-Hexyldecanoic acid (5.2 g, 20 mmol) was dissolved in dichloromethane (20 mL). Diol 8a (1.5 g, 10 mmol), EDC·HCl (2.9 g, 15 mmol), and DMAP (0.5 g, 4 mmol) were added. The mixture was stirred at room temperature for 16-20 h, and the reaction was completed as detected by HPLC. The reaction solution was concentrated at 40 °C until no liquid was distilled out. n-Hexane was added to dissolve the residue, and the mixture was washed twice with acetonitrile. The n-hexane phase was taken and concentrated until no liquid was distilled out. The n-hexane phase was separated by column chromatography to give compound 8b (3.8 g, pale yellow oil, yield: 61%).

### Step 2: Synthesis of compound 8c

Compound 8b (2.5 g, 4 mmol) was dissolved in ethanol (30 mL), and a 2-methylpyridine borane complex (0.97 g, 8 mmol), N,N-dimethyl-1,3-propanediamine (0.41 g, 4 mmol), and acetic acid (0.3 g) were added. The mixture was reacted at room temperature for 24 h. After completion of the reaction, the reaction solution was concentrated until no liquid was distilled out. Ethyl acetate was added, and after achieving complete dissolution, the mixture was washed once with a 10% aqueous hydrochloric acid solution, once with a saturated aqueous sodium sulfite solution, and once with purified water. The organic phase was dried over anhydrous sodium sulfate and concentrated. The obtained oil was dissolved in a methanol:isopropanol (1:4) mixed solution. 5% palladium on carbon (0.5 g) was added, and the mixture was stirred at room temperature for 8 h. The resulting mixture was filtered through celite, and the filtrate was concentrated and separated by column chromatography to give compound 8c (1.5 g, pale yellow oil, yield: 54%).

### Step 3: Synthesis of compound 8

Compound 1e (8.0 g) was dissolved in dichloromethane. Compound 8c (1.4 g, 2.0 mmol) and DMAP (0.4 g, 3.0 mmol) were added. The mixture was stirred at room temperature for 16-20 h, and the reaction was completed as detected by CAD. The reaction solution was concentrated at 40 °C until no liquid was distilled out. n-Hexane was added to dissolve the residue, and the mixture was washed twice with acetonitrile under stirring. The n-hexane phase was concentrated to dryness and separated by column chromatography to give compound 8 (0.79 g, pale yellow oil, yield: 35%).
MS m/z (ESI): 1122.0 [M+1].
1H NMR (300MHz, CDCl₃): δ 5.45 (t, 1H, J = 5.4 Hz), 4.53-4.46 (m, 1H), 4.12 (t, 4H, J = 5.7 Hz), 3.41-3.30 (m, 1H), 3.21-3.10 (m, 2H), 2.54-2.16 (m, 16H), 2.08-0.88 (m, 104H), 0.70 (s, 3H)

### Example 9: Synthesis of Compound 9

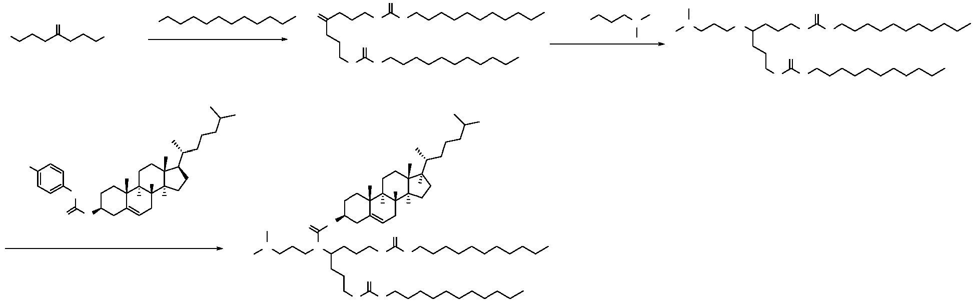

### Step 1: Synthesis of compound 9a

Diol 8a (1.5 g, 10 mmol) was dissolved in 30 mL of dichloromethane, and 4-dimethylaminopyridine (2.5 g, 20 mmol) was added. Subsequently, p-nitrophenyl chloroformate (4.4 g, 22 mmol) was added in batches. The reaction mixture was stirred at room temperature for 3 h. Undecanol (3.5 g, 20 mmol) was added to this reaction solution. The mixture was stirred at room temperature overnight. After the reaction was completed as shown by TLC, the mixture was diluted with 20 mL of dichloromethane and subsequently washed with 30 mL of saturated brine. The organic phase was dried over anhydrous sodium sulfate, filtered, and concentrated to give compound 9a (3.3 g, pale yellow oil, yield: 62%).

### Step 2: Synthesis of compound 9b

Compound 9a (2.2 g, 4 mmol) was dissolved in ethanol (30 mL), and a 2-methylpyridine borane complex (0.97 g, 8 mmol), N,N-dimethyl-1,3-propanediamine (0.41 g, 4 mmol), and acetic acid (0.3 g) were added. The mixture was reacted at room temperature for 24 h. After completion of the reaction, the reaction solution was concentrated until no liquid was distilled out. Ethyl acetate was added, and after achieving complete dissolution, the mixture was washed once with a 10% aqueous hydrochloric acid solution, once with a saturated aqueous sodium sulfite solution, and once with purified water. The organic phase was dried over anhydrous sodium sulfate and concentrated. The obtained oil was dissolved in a methanol:isopropanol (1:4) mixed solution. 5% palladium on carbon (0.5 g) was added, and the mixture was stirred at room temperature for 8 h. The resulting mixture was filtered through celite, and the filtrate was concentrated and separated by column chromatography to give compound 9b (1.3 g, pale yellow oil, yield: 51%).

### Step 3: Synthesis of compound 9

Compound 1e (8.0 g) was dissolved in dichloromethane. Compound 9b (1.3 g, 2.0 mmol) and DMAP (0.4 g, 3.0 mmol) were added. The mixture was stirred at room temperature for 16-20 h, and the reaction was completed as detected by CAD. The reaction solution was concentrated at 40 °C until no liquid was distilled out. n-Hexane was added to dissolve the residue, and the mixture was washed twice with acetonitrile under stirring. The n-hexane phase was concentrated to dryness and separated by column chromatography to give compound 9 (0.78 g, pale yellow oil, yield: 37%).
MS m/z (ESI): 1041.9 [M+1].
1H NMR (300MHz, CDCl₃): δ 5.45 (t, 1H, J = 5.4 Hz), 4.53-4.46 (m, 1H), 4.12 (t, 8H, J = 5.4 Hz), 3.41-3.30 (m, 1H), 3.21-3.10 (m, 2H), 2.54-2.16 (m, 14H), 2.08-0.88 (m, 86H), 0.70 (s, 3H)

### Example 10: Synthesis of Compound 10

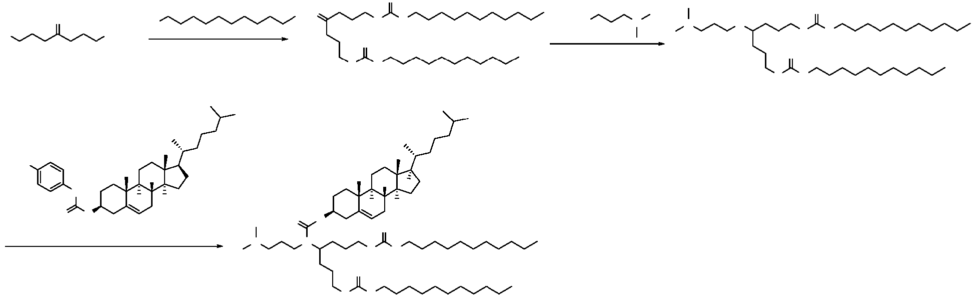

### Step 1: Synthesis of compound 10a

Diol 8a (1.5 g, 10 mmol) was dissolved in 30 mL of dichloromethane, and 4-dimethylaminopyridine (2.5 g, 20 mmol) was added. Subsequently, p-nitrophenyl chloroformate (4.4 g, 22 mmol) was added in batches. The reaction mixture was stirred at room temperature for 3 h. Undecylamine (3.4 g, 20 mmol) was added to this reaction solution. The mixture was stirred at room temperature overnight. After the reaction was completed as shown by TLC, the mixture was diluted with 20 mL of dichloromethane and subsequently washed with 30 mL of saturated brine. The organic phase was dried over anhydrous sodium sulfate, filtered, and concentrated to give compound 10a (3.5 g, pale yellow oil, yield: 65%).

### Step 2: Synthesis of compound 10b

Compound 10a (2.5 g, 4 mmol) was dissolved in ethanol (30 mL), and a 2-methylpyridine borane complex (0.97 g, 8 mmol), N,N-dimethyl-1,3-propanediamine (0.41 g, 4 mmol), and acetic acid (0.3 g) were added. The mixture was reacted at room temperature for 24 h. After completion of the reaction, the reaction solution was concentrated until no liquid was distilled out. Ethyl acetate was added, and after achieving complete dissolution, the mixture was washed once with a 10% aqueous hydrochloric acid solution, once with a saturated aqueous sodium sulfite solution, and once with purified water. The organic phase was dried over anhydrous sodium sulfate and concentrated. The obtained oil was dissolved in a methanol:isopropanol (1:4) mixed solution. 5% palladium on carbon (0.5 g) was added, and the mixture was stirred at room temperature for 8 h. The resulting mixture was filtered through celite, and the filtrate was concentrated and separated by column chromatography to give compound 10b (1.3 g, pale yellow oil, yield: 52%).

### Step 3: Synthesis of compound 10

Compound 1e (8.0 g) was dissolved in dichloromethane. Compound 10b (1.2 g, 2.0 mmol) and DMAP (0.4 g, 3.0 mmol) were added. The mixture was stirred at room temperature for 16-20 h, and the reaction was completed as detected by CAD. The reaction solution was concentrated at 40 °C until no liquid was distilled out. n-Hexane was added to dissolve the residue, and the mixture was washed twice with acetonitrile under stirring. The n-hexane phase was concentrated to dryness and separated by column chromatography to give compound 10 (0.73 g, pale yellow oil, yield: 35%).
MS m/z (ESI): 1039.9 [M+1].
1H NMR (300MHz, CDCl₃): δ 6.85 (s, 2H), 5.45 (t, 1H, J = 5.4 Hz), 4.53-4.46 (m, 1H), 3.98 (t, 4H, J = 5.7 Hz), 3.41-3.30 (m, 1H), 3.26 (t, 4H, J = 5.7 Hz), 3.21-3.10 (m, 2H), 2.54-2.16 (m, 14H), 2.08-0.88 (m, 86H), 0.70 (s, 3H)

### Example 11: Synthesis of Compound 11

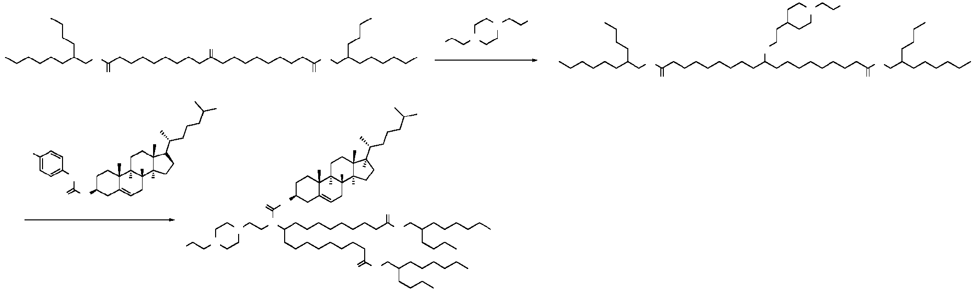

### Step 1: Synthesis of compound 11a

Compound 1c (5.0 g, 7.36 mmol) was dissolved in ethanol (50 mL), and a 2-methylpyridine borane complex (1.57 g, 14.72 mmol), 2-(4-(2-aminoethyl)piperazin-1-yl)ethanol (1.28 g, 7.36 mmol), and acetic acid (0.5 g) were added. The mixture was reacted at room temperature for 24 h. After completion of the reaction, the reaction solution was concentrated until no liquid was distilled out. Ethyl acetate was added, and after achieving complete dissolution, the mixture was washed once with a 10% aqueous hydrochloric acid solution, once with a saturated aqueous sodium sulfite solution, and once with purified water. The organic phase was dried over anhydrous sodium sulfate and concentrated. The obtained oil was dissolved in a methanol:isopropanol (1:4) mixed solution. 5% palladium on carbon (1 g) was added, and the mixture was stirred at room temperature for 8 h. The resulting mixture was filtered through celite, and the filtrate was concentrated and separated by column chromatography to give compound 11a (3.3 g, pale yellow oil, yield: 54%).

### Step 2: Synthesis of compound 11

Compound 1e (13.0 g) was dissolved in dichloromethane. Compound 11a (3.3 g, 4.0 mmol) and DMAP (0.6 g, 5.2 mmol) were added. The mixture was stirred at room temperature for 16-20 h, and the reaction was completed as detected by CAD. The reaction solution was concentrated at 40 °C until no liquid was distilled out. n-Hexane was added to dissolve the residue, and the mixture was washed twice with acetonitrile under stirring. The n-hexane phase was concentrated to dryness and separated by column chromatography to give compound 11 (0.89 g, pale yellow oil, yield: 18%).
MS m/z (ESI): 1249.1 [M+1].
1H NMR (300MHz, CDCl₃): δ 5.45 (t, 1H, J = 5.4 Hz), 4.53-4.46 (m, 1H), 3.98 (d, 4H, J = 5.7 Hz), 3.41-3.33 (m, 3H), 3.21-3.10 (m, 2H), 2.64-2.56 (m, 4H), 2.35-2.12 (m, 14H), 2.08-0.88 (m, 113H), 0.70 (s, 3H)

### Example 12: Preparation and Characterization of mRNA-LNP

The lipid compound of the present disclosure, neutral lipid (phospholipid), and PEG lipid were dissolved in an ethanol solution according to a specific molar ratio (detailed formulation shown in Table 1). The mRNA was dissolved in a citric acid buffer (pH 4.0) to a final concentration of 135 ng/µL. The LNP mixture and the mRNA dilution were mixed at a volume ratio of 1:3, and mRNA-LNP complexes were prepared using a microfluidic nano-preparation system with a flow rate ratio of 1:3 between the LNP mixture and the mRNA dilution, that is, the LNP volume:the mRNA volume was 1:3. After dilution with a 10× phosphate buffer solution (pH 7.4), ultrafiltration concentration was performed, followed by about 500-fold dilution with solution replacement. An experimental sample was obtained after sterile filtration. Samples were taken and analyzed for average particle size, PDI, and Zeta potential.

**Table 1: Three-component LNP lipid formulation based on cholesterol cationic lipid compound**

| No. | Lipid formulation (molar percentage) | Nitrogen-to-phosphorus ratio |
|---|---|---|
| LNP1 | Compound 1:DSPC:DMG-PEG2000 = 49.25:49.25:1.5 | 6 |
| LNP2 | Compound 2:DSPC:DMG-PEG2000 = 49.25:49.25:1.5 | 6 |
| LNP3 | Compound 3:DSPC:DMG-PEG2000 = 49.25:49.25:1.5 | 6 |
| LNP4 | Compound 4:DSPC:DMG-PEG2000 = 49.25:49.25:1.5 | 6 |
| LNP5 | Compound 5:DSPC:DMG-PEG2000 = 49.25:49.25:1.5 | 6 |
| LNP6 | Compound 6:DSPC:DMG-PEG2000 = 49.25:49.25:1.5 | 6 |
| LNP7 | Compound 7:DSPC:DMG-PEG2000 = 49.25:49.25:1.5 | 6 |
| LNP8 | Compound 8:DSPC:DMG-PEG2000 = 49.25:49.25:1.5 | 6 |
| LNP9 | Compound 9:DSPC:DMG-PEG2000 = 49.25:49.25:1.5 | 6 |
| LNP10 | Compound 10:DSPC:DMG-PEG2000 = 49.25:49.25:1.5 | 6 |
| LNP11 | Compound 11:DSPC:DMG-PEG2000 = 49.25:49.25:1.5 | 6 |
| LNP12 | Compound SM-102:DSPC:DMG-PEG2000 = 49.25:49.25:1.5 | 6 |
| LNP13 | Lipid 9::DSPC:DMG-PEG2000=49.25:49.25:1.5 | 6 |

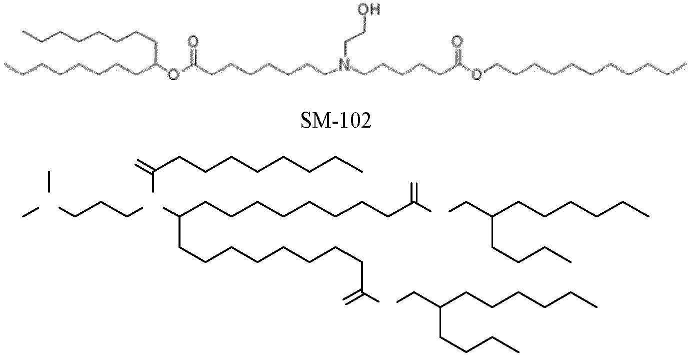

Lipid 9 (prepared by JenKem Technology)

**Example 13:** Preparation, Characterization, and *In Vitro* Cell Transfection Activity Evaluation of Luc-mRNA-LNP Using luciferase gene Luc-mRNA and referring to the preparation method provided in Example 12 and the lipid formulation provided in Table 1, Luc-mRNA-LNP was constructed, whose encapsulation efficiency, average particle size, PDI, and zeta potential data are shown in Table 2. The results demonstrated that the three-component LNP constructed using the cationic lipid compound, together with the phospholipid and PEG lipid provided by the present disclosure, can effectively encapsulate the mRNA, with particle sizes of 80-120 nm, PDI values all less than 0.2, and mRNA encapsulation efficiency higher than 80%. Under the LNP formulation provided by the present disclosure, the commercial lipid SM-102 and other disclosed compounds failed to effectively encapsulate the mRNA, with encapsulation efficiency below 15%.

**Table 2: Physicochemical parameters of Luc-mRNA-LNP**

| No. | Average particle size (nm) | PDI | Zeta potential (mV) | Encapsulation efficiency (%) |
|---|---|---|---|---|
| LNP1 | 116.5 | 0.09924 | -4.558 | 95.21 |
| LNP2 | 105.2 | 0.1247 | -3.251 | 90.11 |
| LNP3 | 105.5 | 0.1117 | -2.626 | 91.27 |
| LNP4 | 104.7 | 0.1606 | -9.922 | 90.61 |
| LNP5 | 91.79 | 0.1469 | 8.847 | 97.55 |
| LNP6 | 83.6 | 0.1598 | 3.219 | 84.70 |
| LNP7 | 96.72 | 0.1762 | 4.836 | 87.92 |
| LNP8 | 108.19 | 0.1471 | 3.417 | 90.86 |
| LNP9 | 105.99 | 0.09241 | 3.46 | 95.34 |
| LNP10 | 98.78 | 0.1311 | 5.99 | 87.16 |
| LNP11 | 92.8 | 0.1671 | 7.542 | 92.28 |
| LNP12 | 82.33 | 0.1193 | -3.5855 | 0.89 |
| LNP13 | 136.5 | 0.1587 | -8.217 | 14.16 |

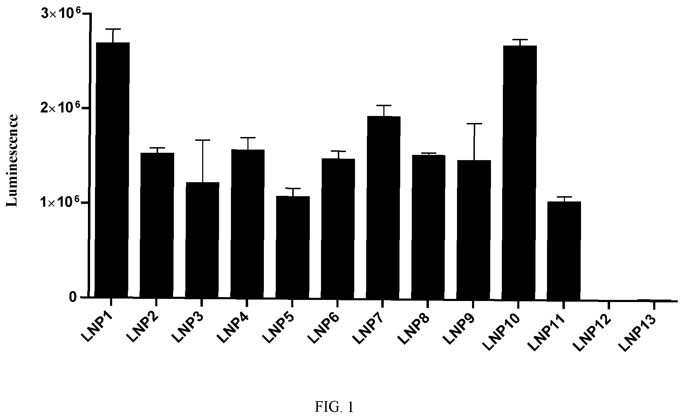

HEK293T cells at 6.5 × 10⁵ cells/mL were seeded into a 96-well cell culture plate at 100 µL/well. After 24 h, cells in each well were transfected with 100 ng of Luc-mRNA-LNP, and the cell culture plate was cultured in a cell incubator at 37 °C with 5% CO₂. The negative control was transfected with an equivalent volume of normal saline. After culturing, bioluminescence detection was performed according to the instructions of the Fire-Lumi^{™} luciferase detection kit, as shown in FIG. 1. The results demonstrated that the three-component LNP constructed using the cationic lipid compound of the present disclosure exhibited superior cell transfection efficiency, enabling high intracellular expression of Luc-mRNA, with expression levels significantly higher than those of the control checks.

### Example 14: mRNA-LNP Animal Immunization Experiment

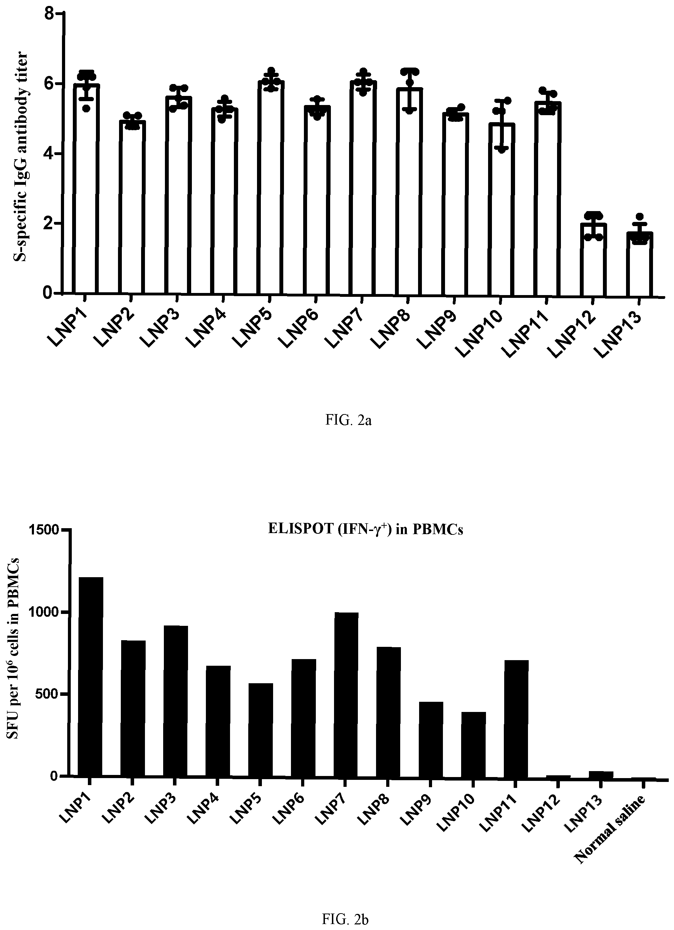

SARS-CoV-2 S protein mRNA was used to evaluate the immune activity of mRNA-LNP in mice, with the LNP formulation referenced in Table 1. The SARS-CoV-2 antigen S protein mRNA-LNP was prepared according to the preparation method provided in **Example 12.** Female BALB/c mice aged 6-8 weeks were randomly divided into groups with 5 mice per group, and immunized via intramuscular injection in the hind leg. Immunization was performed on day 0 and day 14, separately, with an immunization dose of 5 µg mRNA-LNP per mouse. On day 28 post-immunization, blood was collected and serum was separated, and spleen tissues were harvested after euthanasia of the mice. Specific IgG antibody titers against the SARS-CoV-2 S protein antigen encoded by the mRNA were detected by ELISA. PBMCs were isolated and pooled for IFN-γ + cell ELISPOT assay, with results shown in FIGs. 2a and 2b. The LNP delivery system constructed using the cationic lipid compound provided by the present disclosure induced higher antigen-specific antibody titers and stronger cellular immunity when delivering SARS-CoV-2 antigen mRNA in mice compared with the prior art, demonstrating superior efficacy.

Finally, it should be noted that, the above examples are only used to illustrate the technical solutions of the present disclosure, but not to limit the present disclosure. Although the present disclosure is described in detail with reference to the examples described above, it will be understood by those skilled in the art that, modifications may still be made to the technical solutions in the examples described above, or equivalent substitutions may be applied to some or all of the technical features; and these modifications or substitutions do not make the essence of the corresponding technical solutions depart from the scope of the technical solutions in the examples of the present disclosure.

## Claims

1. A lipid compound of formula (I), 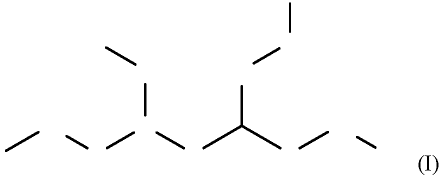
or a stereoisomer thereof, a tautomer thereof, and a pharmaceutically acceptable salt thereof, wherein
L₁, L₂, and L₃ are independently selected from: a chemical bond (-), -O-, -O(C=O)O-, -(C=O)NRa-, -NRa(C=O)-, - NRa-, -O(C=O)-, -(C=O)O-, -C(=O)-, -S(O)x-, -OS(O)xO-, -S-S-, -C(=O)S-, -SC(=O)-, -NRaC(=O)NRa-, - OC(=O)NRa-, -NRaC(=O)O-, -OC(=O)S-, -SC(=O)O-, -P(O)(ORa)O-, and -OP(O)(ORa)O-, wherein x is selected from 0, 1, and 2; Ra is H or C₁-C₁₂ hydrocarbyl;
G₁, G₂, G₃, G₄, and G₅ are each independently selected from: a chemical bond (-), -C₁-C₂₄ alkylene, -C₂-C₂₄ alkenylene, -C₃-C₈ cycloalkylene, and -C₃-C₈ cycloalkenylene;
R₁ is selected from H, -OR₅, -NR₅R₆, -NR₅C(=O)R₆, -C(=O)OR₅, -OC(=O)R₅, -CN, -R₈, and 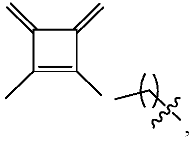 wherein R₅ and R₆ are each independently selected from H, -C₁-C₁₂ alkyl, -C₂-C₁₂ alkenyl, -C₂-C₁₂ alkynyl, -C₁-C₁₂ alkoxy, -C₃-C₈ cycloalkyl, -C₃-C₈ cycloalkenyl, and -C₃-C₈ cycloalkynyl; R₇ is -NR₇'R₇", and R₇' and R₇" are each independently selected from C₁-C₆ alkyl, C₂-C₃ alkenyl, and H; R₈ is selected from pyrazole, imidazole, piperidine, pyrrole, pyrrolidine, piperazine, indole, purine, and morpholine, and the group is optionally substituted with C₁-C₆ alkyl, C₁-C₆ alkenyl, halogen, and hydroxy; n is an integer selected from 1-10; and R₂, R₃, and R₄ are each independently selected from a naturally occurring or non-naturally occurring steroid group, C₆-C₂₄ alkyl, C₆-C₂₄ alkenyl, and C₆-C₂₄ alkynyl,
provided that at least one of R₂, R₃, and R₄ is a naturally occurring or non-naturally occurring steroid group.

2. The compound, or the stereoisomer thereof, the tautomer thereof, and the pharmaceutically acceptable salt thereof according to claim 1, wherein,
L₁, L₂, and L₃ are each independently selected from: -O(C=O)O-, -O(C=O)-, -(C=O)O-, -C(=O)-, -(C=O)NRₐ-, - NRₐ(C=O)-, -OC(=O)NRₐ-, -NRₐC(=O)O-, -S(O)ₓ-, -SC(=O)-, and -C(=O)S-, wherein x is selected from 0, 1, and 2; Ra is H or C₁-C₁₂ hydrocarbyl;
G₁, G₂, G₃, G₄, and G₅ are each independently selected from: a chemical bond (-), -C₁₋₁₈ alkylene, -C₂₋₁₈ alkenylene, -C₃₋₈ cycloalkylene, and -C₃₋₈ cycloalkenylene; and
R₁ is selected from H, -OR₅, -NR₅R₆, and -R₈, wherein R₅ and R₆ are each independently selected from H, -C₁-C₁₂ alkyl, -C₂-C₁₂ alkenyl, -C₂-C₁₂ alkynyl, -C₁-C₁₂ alkoxy, -C₃-C₈ cycloalkyl, -C₃-C₈ cycloalkenyl; R₈ is selected from pyrazole, imidazole, pyrrole, pyrrolidine, piperidine, and methylpiperidine.

3. The compound, or the stereoisomer thereof, the tautomer thereof, and the pharmaceutically acceptable salt thereof according to claim 1 or 1, wherein,
L₁, L₂, and L₃ are each independently selected from: -O(C=O)O-, -O(C=O)-, -(C=O)O-, -C(=O)-, -C(=O)S-, - SC(=O)-, -NHC(=O)O-, and -OC(=O)NH-;
G₁, G₂, G₃, G₄, and G₅ are each independently selected from: a chemical bond (-) and -C₁-C₁₀ alkylene; and
R₁ is selected from -OR₅, -NR₅R₆, and -R₈, wherein R₅ and R₆ are each independently selected from H and -C₁-C₅ alkyl; R₈ is selected from pyrazole, imidazole, pyrrole, pyrrolidine, piperidine, and methylpiperidine.

4. The compound, or the stereoisomer thereof, the tautomer thereof, and the pharmaceutically acceptable salt thereof according to any one of claims 1-3, wherein the steroid is selected from: avenasterol, β-sitosterol, brassicasterol, ergocalciferol, campesterol, cholestanol, cholesterol, an oxidized form of cholesterol, a reduced form of cholesterol, coprostanol, dehydrocholesterol, desmosterol, dihydroergocalciferol, dihydrocholesterol, dihydroergosterol, dinosterol, epicholesterol, ergosterol, fucosterol, hexahydrolumisterol, hydroxycholesterol, lanosterol, lumisterol, saringosterol, sitostanol, sitosterol, stigmastanol, stigmasterol, cholic acid, glycocholic acid, taurocholic acid, deoxycholic acid, lithocholic acid, and/or alkyl lithocholate.

5. The compound, or the stereoisomer thereof, the tautomer thereof, and the pharmaceutically acceptable salt thereof according to any one of the preceding claims, wherein the steroid group is selected from: 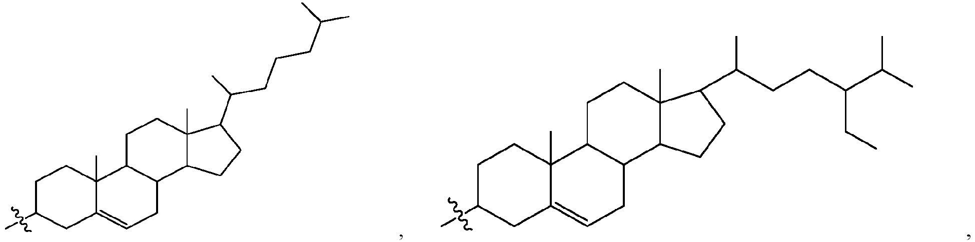 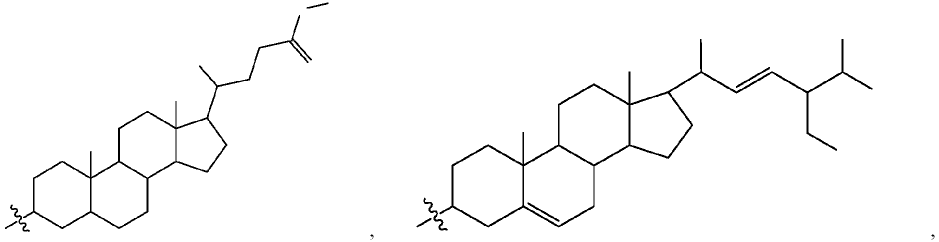 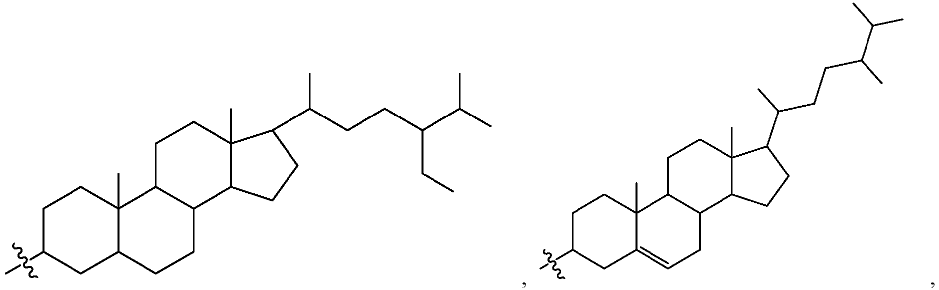 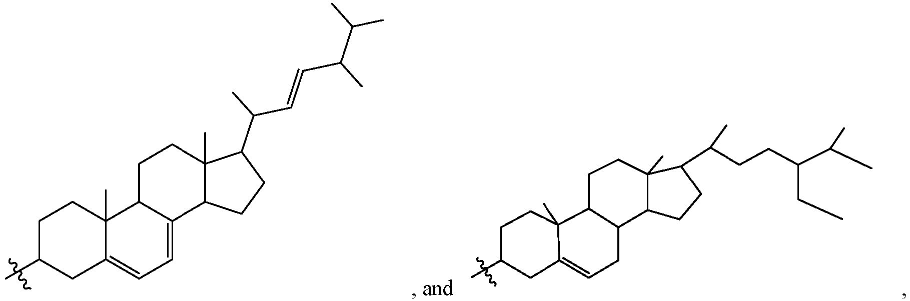 wherein R is C₁₋₂₀ alkyl.

6. The compound according to any one of the preceding claims, wherein the compound is a compound of formula (II), 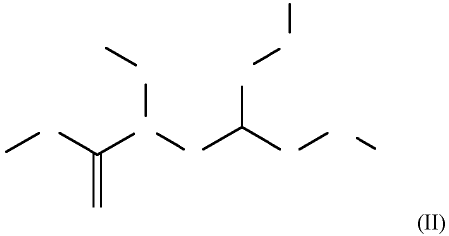 or a stereoisomer thereof, a tautomer thereof, and a pharmaceutically acceptable salt thereof, wherein L₂, L₃, G₁, G₂, G₃, G₄, R₁, R₂, R₃, and R₄ are as defined in the preceding claims.

7. The compound according to any one of the preceding claims, wherein the compound is a compound of formula (III), 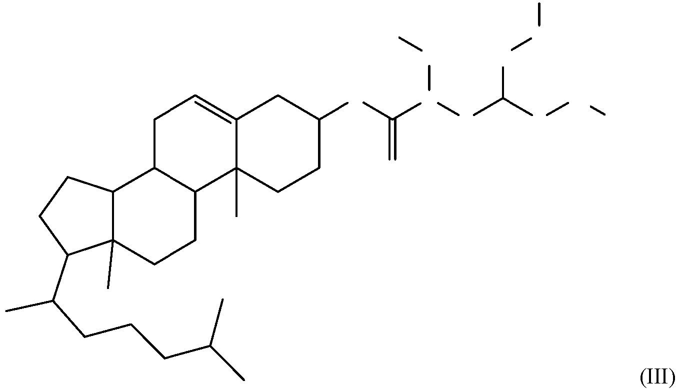 or a stereoisomer thereof, a tautomer thereof, and a pharmaceutically acceptable salt thereof, wherein L₂, L₃, G₁, G₂, G₃, G₄, R₁, R₂, and R₃ are as defined in the preceding claims.

8. The compound according to any one of the preceding claims, wherein the compound is a compound of formula (IV), 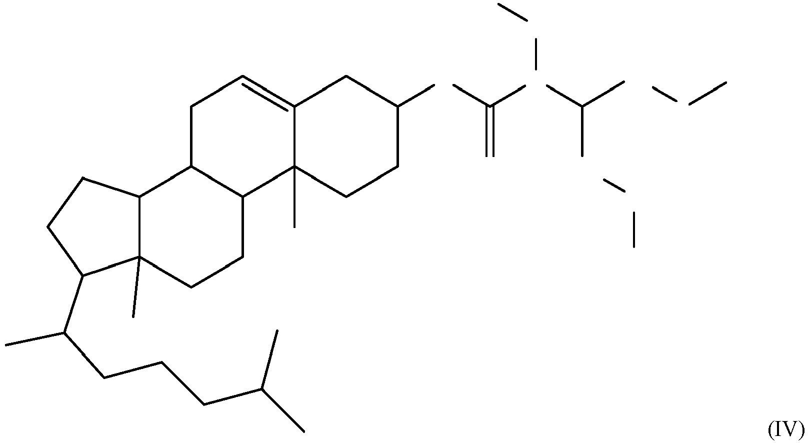 or a stereoisomer thereof, a tautomer thereof, and a pharmaceutically acceptable salt thereof, wherein L₂, L₃, G₂, G₃, G₄, R₁, R₂, and R₃ are as defined in the preceding claims.

9. The compound according to any one of the preceding claims, wherein the compound is a compound of formula (V), 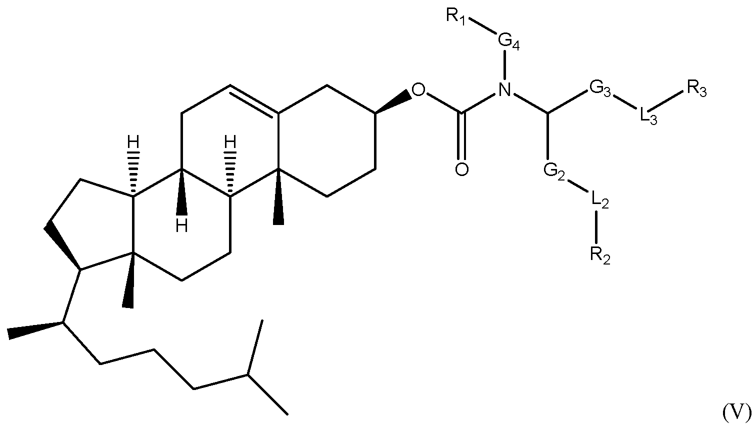 or a stereoisomer thereof, a tautomer thereof, and a pharmaceutically acceptable salt thereof, wherein L₂, L₃, G₂, G₃, G₄, R₁, R₂, and R₃ are as defined in the preceding claims.

10. The lipid compound, or the stereoisomer thereof, the tautomer thereof, and the pharmaceutically acceptable salt thereof according to any one of the preceding claims, wherein
L₂ and L₃ are each independently selected from: -O(C=O)O-, -O(C=O)-, -(C=O)O-, -C(=O)-, -C(=O)S-, -SC(=O)-, -NHC(=O)O-, and -OC(=O)NH-;
G₂, G₃, and G₄ are each independently selected from -C₁-C₁₀ alkylene;
R₁ is selected from -OR₅, -NR₅R₆, and -R₈, wherein R₅ and R₆ are each independently selected from H and -C₁-C₅ alkyl; R₈ is selected from pyrazole, imidazole, pyrrole, pyrrolidine, piperidine, and methylpiperidine;
R₂ and R₃ are each independently selected from: 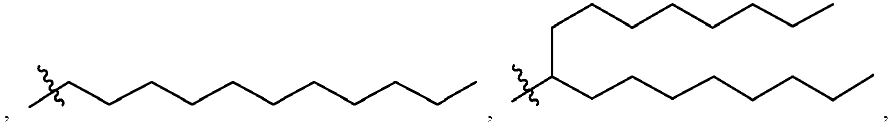 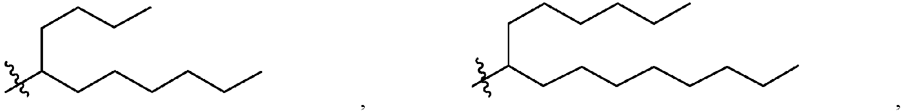 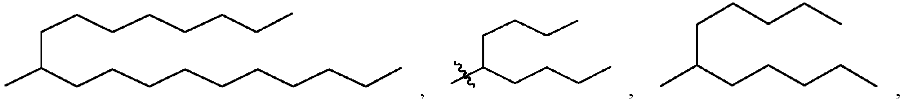 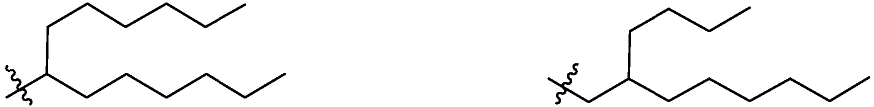 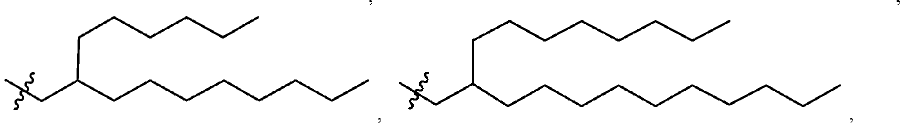 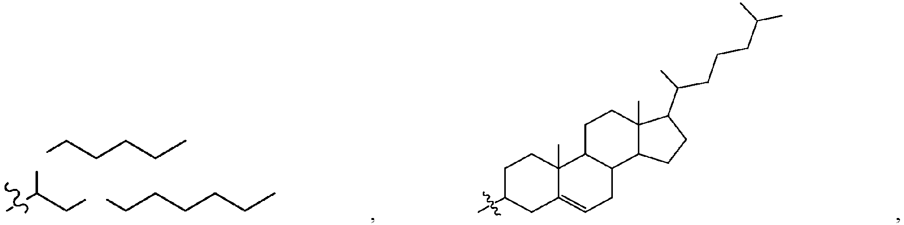 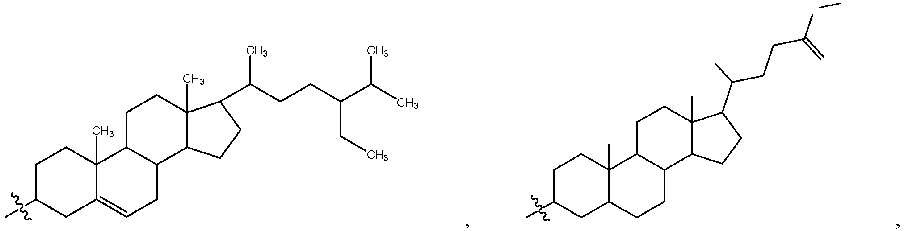 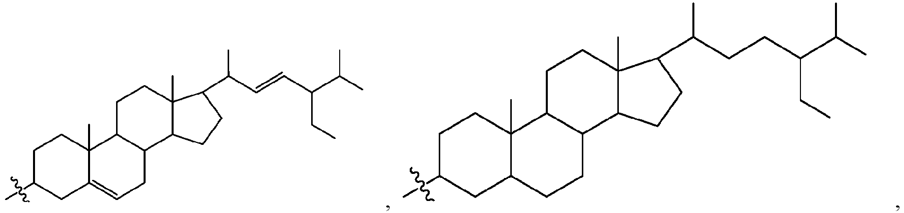 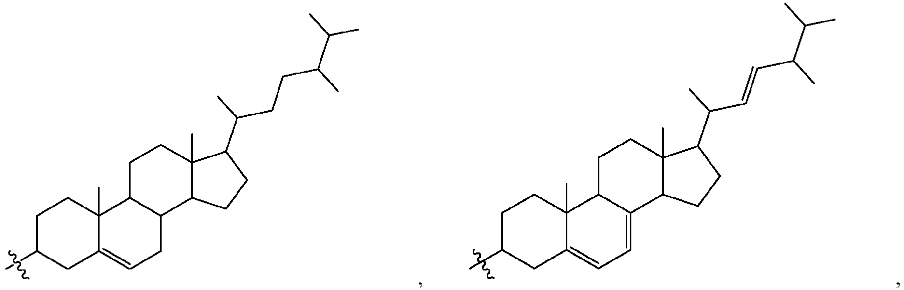 and 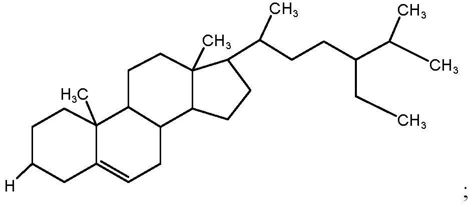 and
preferably, R₂ and R₃ are each independently selected from: 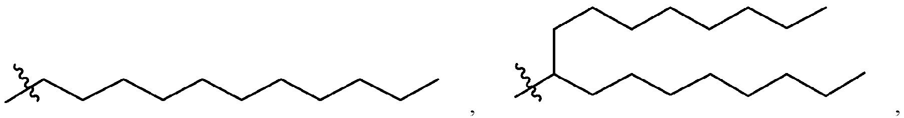 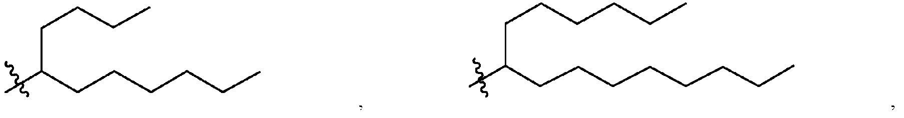 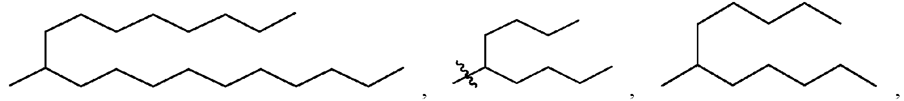 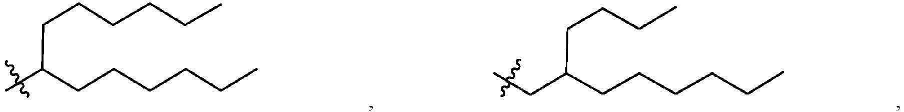 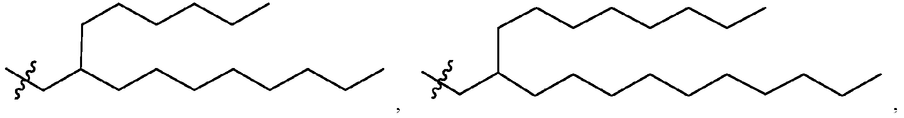 , and

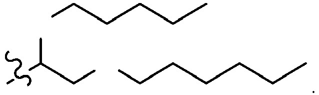

11. The compound according to claim 1, wherein the compound is selected from: 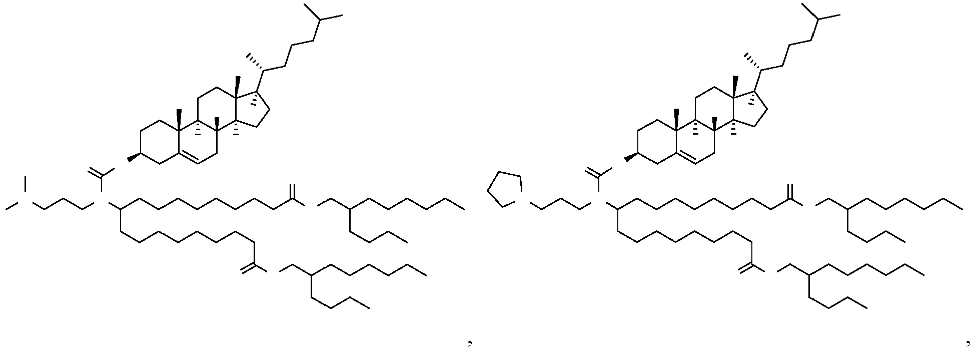 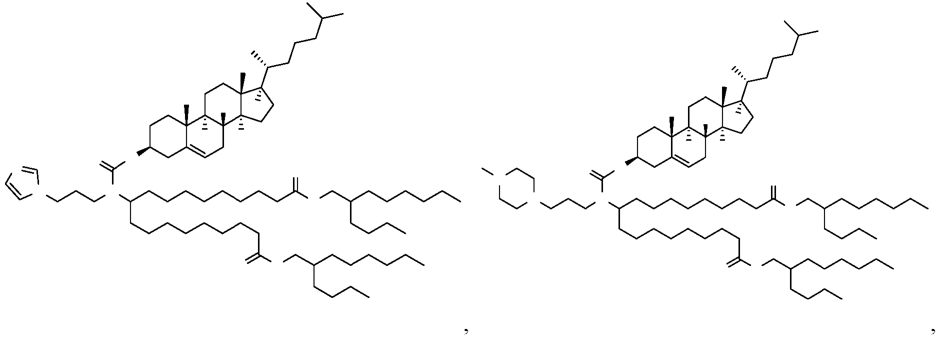 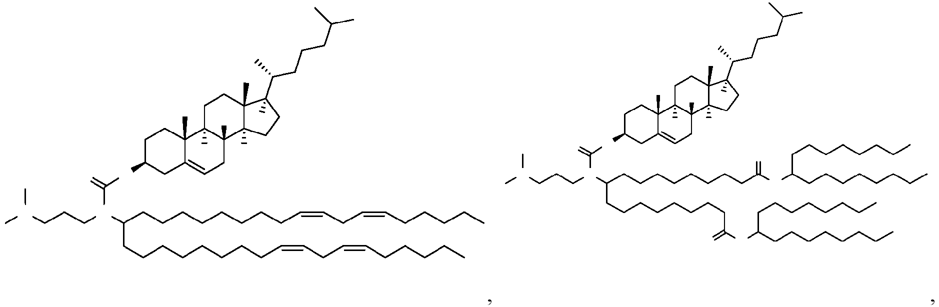 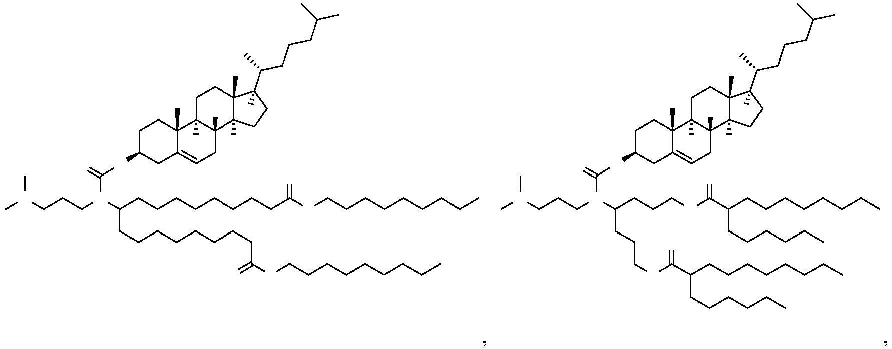 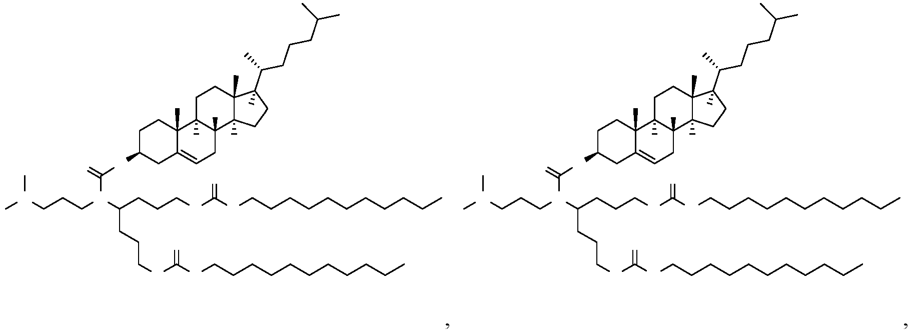 and 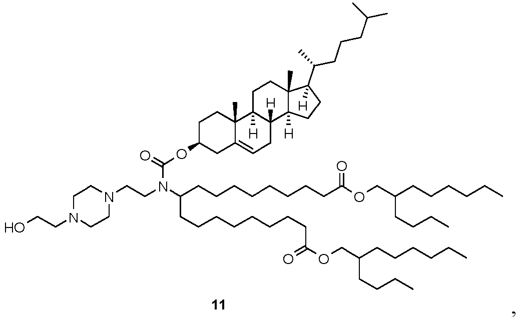 or a stereoisomer thereof, a tautomer thereof, and a pharmaceutically acceptable salt thereof.

12. A lipid nanoparticle (LNP), comprising the lipid compound according to any one of the preceding claims.

13. The LNP according to claim 12, further comprising a polyethylene glycol lipid and at least one second lipid, wherein the second lipid is selected from: a neutral lipid, a zwitterionic lipid, and an anionic lipid.

14. The LNP according to claim 13, wherein the polyethylene glycol lipid is selected from: 2-[(polyethylene glycol)-2000]-N,N-ditetradecylacetamide, 1,2-dimyristoyl-sn-glycerol methoxypolyethylene glycol, 1,2-distearoyl-sn-glycero-3-phosphoethanolamine-N-[amino(polyethylene glycol)], PEG-disterol glycerol, PEG-dipalmitoyl, PEG-dioleyl, PEG-distearyl, PEG-diacylglycerol amide, PEG-dipalmitoyl phosphatidylethanolamine, and PEG-1,2-dimyristoyloxypropyl-3-amine; and
the neutral lipid is selected from: 1,2-distearoyl-sn-glycero-3-phosphocholine, 1,2-dipalmitoyl-sn-glycero-3-phosphocholine, 1,2-dioleoyl-sn-glycero-3-phosphoethanolamine, 1,2-dipalmitoyl-sn-glycero-3-phosphoethanolamine, 1,2-dimyristoyl-sn-glycero-3-phosphoethanolamine (DMPE), 2-dioleoyl-sn-glycero-3-phospho-(1'-rac-glycerol), oleoyl phosphatidylcholine, and 1-palmitoyl-2-oleoyl phosphatidylethanolamine.

15. Use of the lipid compound according to claims 1-11 and/or the LNP according to any one of claims 12-14 in preparing a bioactive substance delivery system.

16. The use according to claim 15, wherein the bioactive substance is a DNA or an RNA, and the RNA is selected from an antisense RNA, an saRNA, an mRNA, an lncRNA, an miRNA, an siRNA, a piRNA, a gRNA, a tsRNA, a circRNA, and a self-replicating mRNA.

17. The use according to claim 16, wherein the bioactive substance delivery system is an mRNA vaccine for preventing cancer, a viral infection, a bacterial infection, and a fungal infection; preferably, the virus is selected from: norovirus, Ebola virus, coronavirus, cytomegalovirus, Dengue virus, Zika virus, coxsackievirus, enterovirus, hepatitis virus, herpes simplex virus, human papilloma virus, influenza virus, Marburg virus, measles virus, poliovirus, rabies virus, rotavirus, and measles virus.

18. A medicament, comprising the bioactive substance according to claim 16 and the LNP according to any one of claims 12-14, and a pharmaceutically acceptable auxiliary material.

19. The medicament according to claim 18, wherein the medicament is a gene drug.
